# EUROPEAN PATENT APPLICATION

(11) **EP 4 606 384 A1**
(43) Date of publication of application: **27.08.2025**
(21) Application number: 23879809.4
(22) Date of filing: 18.10.2023
(51) Int. Cl.: A61K 39/395, A61K 45/00, A61P 1/00, A61P 11/00, A61P 13/10, A61P 35/00, C07K 16/28, C07K 16/46

(54) **USE OF ANTI-CLDN4-ANTI-CD137 BISPECIFIC ANTIBODY COMBINED WITH PD-1 SIGNAL INHIBITOR FOR CANCER TREATMENT**

(30) Priority: 19.10.2022 JP 2022167362
(71) Applicant: Astellas Pharma, Inc., Chuo-ku Tokyo 103-8411 (JP)
(72) Inventor: SATO, Masahito, Tokyo 103-8411 (JP); HIROUCHI, Keita, Tokyo 103-8411 (JP); KIKUCHI, Aya, Tokyo 103-8411 (JP)
(74) Representative: Hoffmann Eitle
(86) International application number: PCT/JP2023/037616
(87) International publication number: WO 2024/085166

(57) **Abstract**

An object of the present invention is to provide an anti-CLDN4/anti-CD 137 bispecific antibody to be used in combination with a PD-1 signal inhibitor for treating cancer of a subject, a pharmaceutical composition comprising the bispecific antibody, or a method for treating cancer, including administering an anti-CLDN4/anti-CD137 bispecific antibody in combination with a PD-1 signal inhibitor to a subject. Under a coculture condition of CLDN4 expressing cancer cells and T cells, the use of both an anti-CLDN4/anti-CD137 bispecific antibody and a PD-1 signal inhibitor exhibited a promoting activity on production of interferon-y by a T cell and a cytotoxic action, and exhibited a significant antitumor activity in a CLDN4 expressing cancer cell-transplanted mice, as compared with administration of each alone. From the results, it was shown that a combination of the anti-CLDN4/anti-CD137 bispecific antibody and the PD-1 signal inhibitor is useful for treatment of CLDN4 expressing cancer.

## Description

### TECHNICAL FIELD

The present invention relates to use of an anti-CLDN4/anti-CD 137 bispecific antibody in combination with a PD-1 signal inhibitor in cancer treatment.

### BACKGROUND ART

Claudin-4 (CLDN4) is a tetraspan membrane protein belonging to the claudin family. It is expressed in an epithelial cell and an endothelial cell, and plays a significant role as a main molecule comprised in a tight junction. CLDN4 is highly expressed in a cancer tissue of colorectal cancer, bladder cancer, ovarian cancer and the like, and it has been suggested that an anti-CLDN4 antibody is possibly applicable to treatment or diagnosis of cancer (PTL 1 and NPL 1). Besides, it has been described that use of both an anti-CLDN4 antibody and an anti-epidermal growth factor receptor (EGFR) antibody has an antitumor effect in an animal model (NPL 2).

Cluster of differentiation 137 (CD137, another name 4-1BB) is a molecule belonging to the tumor necrosis factor receptor superfamily (TNFRSF), and has been reported to express on a cell surface of an immune cell such as a T cell, a B cell, a natural killer (NK) cell, a dendritic cell, an eosinophil, or a mast cell. In particular, CD137 on a T cell binds to a CD137 ligand on an antigen presenting cell and is known to be involved in activation and survival of the T cell as a costimulatory molecule (NPL 3). An anti-CD137 agonist antibody presents an antitumor effect through activation of an immune cell in a tumor microenvironment in an animal model (NPL 4). Urelumab, that is, an anti-CD137 agonist antibody, presented a therapeutic effect in a clinical trial, but is reported to cause an adverse reaction of liver disorder (NPL 5).

As an innovative method by which cancer cell-selective cytotoxic activity can be obtained at a low antibody concentration, a bispecific T cell recruiting antibody with various antibody formats has been reported. A bispecific T cell recruiting antibody is a bispecific antibody comprising an antibody against a tumor-associated antigen (TAA) expressing on a cancer cell surface, and an antibody binding to a T cell, and is started to be examined for an effect of these antibodies in T cell-mediated immunotherapy (NPL 6). As an antibody binding to a T cell, an anti-CD3 antibody is often used, and bispecific T cell recruiting antibodies to various TAAs are now being studied and developed.

Furthermore, bispecific T cell recruiting antibodies against CD137 and TAA are being earnestly studied in recent years. Studies are being made on an anti-GPC3/anti-CD137 bispecific antibody, an anti-HER2/anti-CD137 bispecific antibody, an anti-PD-L1/anti-CD137 bispecific antibody, an anti-FAP/anti-CD137 bispecific antibody and the like recognizing TAAs of glypican 3 (GPC3), human epidermal growth factor receptor type 2 (HER2), programmed cell-death ligand 1 (PD-L1), and fibroblast activation protein (FAP) (PTLs 2 and 3, and NPLs 7 to 9).

Programmed cell death-1 (PD-1; also designated as PDCD1 or CD279) is a 50-55 kDa type I transmembrane protein belonging to the immunoglobulin superfamily (NPL 10). PD-1, expression of which is induced in a T cell with sustained activation, regulates activation of the T cell in an inhibitory manner by binding to the ligand programmed deathligand 1 (PD-L1; also designated as PDCD1LG1, B7-H1, or CD274) or programmed deathligand 2 (PD-L2; also designated as PDCD1LG2, B7-DC, or CD273) (NPL 11). In general, such a T cell activation regulatory mechanism is designated as an immune checkpoint and known as one of the negative feedback mechanisms to prevent excessive immune responses.

In the early stage of cancer development, an immune cell such as a T cell eliminates cancer by an antitumor immune response via immune surveillance. On the other hand, cancer acquires an immune escape mechanism by directly or indirectly suppressing immune cells in the cancer microenvironment. Immune checkpoint mechanisms such as the PD-1/PD-L1 or PD-L2 (hereinafter the "PD-1 signal") pathway and the CTLA-4/CD80 or CD86 pathway are known as a direct activated T-cell inhibitory mechanism. In the tumor microenvironment of cancer, expression of PD-1 in a T cell and expression of PD-L1 in a tumor have been observed (NPL 12), and the cancer is expected to exhibit immune escape through activation of the PD-1 signal. It has been reported in a plurality of tumor-bearing mouse models that inhibition of PD-1 signals leads to antitumor activity through release of immune escape mechanisms (NPLs 13 to 15). Furthermore, as PD-1 signal inhibitors, development of anti-PD-1 antibodies such as nivolumab and pembrolizumab has been vigorously conducted, and the great results have been achieved in melanoma, lung cancer, lymphoma, or the like. In addition to the antibodies, nucleic acid drugs, small molecule drugs, and the like as PD-1 signal inhibitors are also being studied (NPL 16).

In order to enhance the efficacy of treatment for a cancer patient, combination use therapy with a plurality of immuno-oncology drugs and combination use trials of an immuno-oncology drug and an existing anticancer drug are being vigorously conducted (NPLs 17 and 18). For example, a combination use of an anti-PD-1 antibody with another immune checkpoint blocking antibody, an anticancer drug, a molecular-targeted drug, radiation therapy, a cancer vaccine, or an oncolytic virus is being tested.

There is, until now, however, no report on a method for treating cancer using an anti-CLDN4/anti-CD137 bispecific antibody in combination with a PD-1 signal inhibitor.

### CITATION LIST

### PATENT LITERATURE

PTL 1: WO2008/114733
PTL 2: WO2015/156268
PTL 3: WO2016/177802

### NON PATENT LITERATURE

NPL 1: Cancer Science, 2009: 100(9): pp. 1623-1630
NPL 2: Oncotarget, 2018: 9(100): p.37367-37378
NPL 3: Cancer Science, 2020: 111(5): p.1461-1467
NPL 4: Cancer Immunology Immunotherapy, 2012: 61(5): p.1721-1733
NPL 5: Clinical Cancer Research, 2017: 23(8): p.1929-1936
NPL 6: MAbs, 2017: 9(2): p.182-212
NPL 7: Clinical Cancer Research, 2019: 25(19): p.5878-5889
NPL 8: Clinical Cancer Research, 2020: 26(15): p.4154-4167
NPL 9: Journal for Immunotherapy of Cancer, 2020: 8(2): e000238
NPL 10: International Immunology, 1996: Vol.8: p.765-772
NPL 11: Annual Review of Immunology, 2008: Vol.26: p.677-704
NPL 12: Nature Medicine, 2002:8: p.793-800
NPL 13: Scientific Reports, 2021:11: p.21087-21099
NPL 14: Nature Communications, 2017: 8: p.14572-14582
NPL 15: Journal for Immunotherapy of Cancer, 2019: 7: 37: p.1-16
NPL 16: Molecules, 2019: 24: p.2071-2100
NPL 17: Cancer Discovery, 2021: 11: p.1368-1397
NPL 18: Molecular Medicine Reports, 2021: 23: p.362-377

### SUMMARY OF INVENTION

### TECHNICAL PROBLEM

An object of the present invention is to provide an anti-CLDN4/anti-CD137 bispecific antibody to be used in combination with a PD-1 signal inhibitor for treating cancer of a subject, a pharmaceutical composition comprising the bispecific antibody, or a method for treating cancer, including administering an anti-CLDN4/anti-CD137 bispecific antibody and a PD-1 signal inhibitor to a subject.

### SOLUTION TO PROBLEM

For the purpose of creating an antibody or pharmaceutical composition to be used for the treatment of CLDN4 expressing cancer, the present inventors produced an anti-CLDN4/anti-CD137 bispecific antibody based on the sequences of an antibody KM3900, which is a known anti-CLDN4 antibody, and an anti-CD137 antibody (Example 1). Combination useof the obtained anti-CLDN4/anti-CD137 bispecific antibody and an anti-PD-1 antibody or an anti-PD-L1 antibody promoted production of interferon y of a T cell *in vitro* as compared to the case of using the anti-CLDN4/anti-CD137 bispecific antibody, anti-PD-1 antibody, or anti-PD-L1 antibody alone (Examples 2 and 3). Furthermore, in mice bearing human CLDN4 expressing mouse tumor cells, the combination use of the anti-CLDN4/anti-CD137 bispecific antibody and the anti-PD-1 antibody exhibited a significant antitumor effect compared to administration of either the anti-CLDN4/anti-CD137 bispecific antibody or the anti-PD-1 antibody alone (Example 4). From the results, it has been suggested that a combination of the anti-CLDN4/anti-CD137 bispecific antibody and the PD-1 signal inhibitor is useful for treatment of CLDN4 expressing cancer.

Specifically, the present invention relates to the following [1] to [84], although not limited thereto.
[1] A pharmaceutical composition for treating cancer of a subject, comprising an anti-CLDN4/anti-CD137 bispecific antibody, which is used in combination with a PD-1 signal inhibitor, wherein the bispecific antibody comprises a heavy chain variable region and a light chain variable region of an anti-CLDN4 antibody, and a heavy chain variable region and a light chain variable region of an anti-CD137 antibody.
[2] The pharmaceutical composition according to [1], wherein the heavy chain variable region of the anti-CLDN4 antibody comprises a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and the light chain variable region of the anti-CLDN4 antibody comprises a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4.
[3] The pharmaceutical composition according to [1] or [2], wherein the heavy chain variable region of the anti-CLDN4 antibody consists of an amino acid sequence at amino acid positions 1-123 of SEQ ID NO: 2, and the light chain variable region of the anti-CLDN4 antibody consists of an amino acid sequence at amino acid positions 1-109 of SEQ ID NO: 4.
[4] The pharmaceutical composition according to any of [1] to [3], wherein the anti-CLDN4/anti-CD137 bispecific antibody comprises an IgG antibody (anti-CLDN4 IgG antibody) consisting of a heavy chain comprising the heavy chain variable region of the anti-CLDN4 antibody and a light chain comprising the light chain variable region of the anti-CLDN4 antibody.
[5] The pharmaceutical composition according to [4], comprising either LALA mutation (L234A and L235A) or P331G mutation (wherein a position of the mutation is an amino acid position in human Igγ1 constant region according to EU index) or both in a Fc region of the anti-CLDN4 IgG antibody.
[6] The pharmaceutical composition according to any of [1] to [5], wherein the heavy chain variable region of the anti-CD137 antibody comprises a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and the light chain variable region of the anti-CD 137 antibody comprises a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2.
[7] The pharmaceutical composition according to any of [1] to [6], wherein the heavy chain variable region of the anti-CD137 antibody consists of an amino acid sequence at amino acid positions 595-712 of SEQ ID NO: 2, and the light chain variable region of the anti-CD137 antibody consists of an amino acid sequence at amino acid positions 464-573 of SEQ ID NO: 2.
[8] The pharmaceutical composition according to [6] or [7], wherein the anti-CLDN4/anti-CD137 bispecific antibody comprises an anti-CD137 single chain variable fragment (anti-CD137 scFv) comprising the heavy chain variable region and the light chain variable region of the anti-CD137 antibody.
[9] The pharmaceutical composition according to [8], wherein the anti-CD137 scFv consists of an amino acid sequence at amino acid positions 464-712 of SEQ ID NO: 2.
[10] The pharmaceutical composition according to [8] or [9], wherein the anti-CLDN4/anti-CD137 bispecific antibody comprises an anti-CLDN4 IgG antibody and the anti-CD137 scFv, and an amino terminal of the anti-CD 137 scFv is linked to a heavy chain carboxy terminal of the anti-CLDN4 IgG antibody via a linker.
[11] A pharmaceutical composition for treating cancer of a subject, comprising an anti-CLDN4/anti-CD137 bispecific antibody, which is used in combination with a PD-1 signal inhibitor, wherein the bispecific antibody comprises a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region consisting of an amino acid sequence at amino acid positions 1-109 of SEQ ID NO: 4, and an anti-CD137 scFv comprising a light chain variable region of an anti-CD137 antibody consisting of an amino acid sequence at amino acid positions 464-573 of SEQ ID NO: 2 and a heavy chain variable region of an anti-CD137 antibody consisting of an amino acid sequence at amino acid positions 595-712 of SEQ ID NO: 2, wherein an amino terminal of the anti-CD137 scFv is linked to a heavy chain carboxy terminal of the anti-CLDN4 antibody via a linker.
[12] A pharmaceutical composition for treating cancer of a subject, comprising an anti-CLDN4/anti-CD137 bispecific antibody, which is used in combination with a PD-1 signal inhibitor, wherein the bispecific antibody comprises a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4, and an anti-CD 137 scFv consisting of an amino acid sequence at amino acid positions 464-712 of SEQ ID NO: 2, wherein an amino terminal of the anti-CD137 scFv is linked to a heavy chain carboxy terminal of the anti-CLDN4 antibody via a linker.
[13] The pharmaceutical composition according to any of [10] to [12], wherein the linker is a GS linker.
[14] A pharmaceutical composition for treating cancer of a subject, comprising an anti-CLDN4/anti-CD137 bispecific antibody, which is used in combination with a PD-1 signal inhibitor, wherein the bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4.
[15] The pharmaceutical composition according to any of [1] to [14], wherein the anti-CLDN4/anti-CD137 bispecific antibody is post-translationally modified.
[16] The pharmaceutical composition according to any of [1] to [15], which is used in combination with the PD-1 signal inhibitor simultaneously, continuously, or sequentially.
[17] The pharmaceutical composition according to any of [1] to [16], wherein the anti-CLDN4/anti-CD137 bispecific antibody and the PD-1 signal inhibitor are (i) comprised in the same pharmaceutical composition and administered simultaneously or (ii) comprised in separate pharmaceutical compositions and used in combination simultaneously, continuously, or sequentially.
[18] The pharmaceutical composition according to any of [1] to [17], wherein the cancer is selected from the group consisting of colorectal cancer, bladder cancer, and lung cancer.
[19] The pharmaceutical composition according to any of [1] to [18], wherein the PD-1 signal inhibitor is an antibody binding to one or more proteins selected from the group consisting of PD-1, PD-L1, and PD-L2, or an antigen binding fragment thereof.
[20] The pharmaceutical composition according to any of [1] to [19], wherein the PD-1 signal inhibitor is an anti-PD-1 antibody selected from the group consisting of nivolumab, pembrolizumab, pidilizumab, spartalizumab, and cemiplimab.
[21] The pharmaceutical composition according to any of [1] to [19], wherein the PD-1 signal inhibitor is an anti-PD-1 antibody selected from the group consisting of atezolizumab, durvalumab, and avelumab.
[22] An anti-CLDN4/anti-CD137 bispecific antibody for treating cancer of a subject, comprising a heavy chain variable region and a light chain variable region of an anti-CLDN4 antibody, and a heavy chain variable region and a light chain variable region of an anti-CD137 antibody, wherein the bispecific antibody is used in combination with a PD-1 signal inhibitor.
[23] The bispecific antibody according to [22], wherein the heavy chain variable region of the anti-CLDN4 antibody comprises a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and the light chain variable region of the anti-CLDN4 antibody comprises a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4.
[24] The bispecific antibody according to [22] or [23], wherein the heavy chain variable region of the anti-CLDN4 antibody consists of an amino acid sequence at amino acid positions 1-123 of SEQ ID NO: 2, and the light chain variable region of the anti-CLDN4 antibody consists of an amino acid sequence at amino acid positions 1-109 of SEQ ID NO: 4.
[25] The bispecific antibody according to any of [22] to [24], comprising an IgG antibody (anti-CLDN4 IgG antibody) consisting of a heavy chain comprising the heavy chain variable region of the anti-CLDN4 antibody and a light chain comprising the light chain variable region of the anti-CLDN4 antibody.
[26] The bispecific antibody according to [25], comprising either LALA mutation (L234A and L235A) or P331G mutation (wherein a position of the mutation is an amino acid position in human Igγ1 constant region according to EU index) or both in a Fc region of the anti-CLDN4 IgG antibody.
[27] The bispecific antibody according to any of [22] to [26], wherein the heavy chain variable region of the anti-CD137 antibody comprises a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and the light chain variable region of the anti-CD 137 antibody comprises a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2.
[28] The bispecific antibody according to any of [22] to [27], wherein the heavy chain variable region of the anti-CD137 antibody consists of an amino acid sequence at amino acid positions 595-712 of SEQ ID NO: 2, and the light chain variable region of the anti-CD137 antibody consists of an amino acid sequence at amino acid positions 464-573 of SEQ ID NO: 2.
[29] The bispecific antibody according to [27] or [28], comprising an anti-CD137 single chain variable fragment (anti-CD137 scFv) comprising the heavy chain variable region and the light chain variable region of the anti-CD137 antibody.
[30] The bispecific antibody according to [29], wherein the anti-CD137 scFv consists of an amino acid sequence at amino acid positions 464-712 of SEQ ID NO: 2.
[31] The bispecific antibody according to [29] or [30], comprising an anti-CLDN4 IgG antibody and the anti-CD 137 scFv, wherein an amino terminal of the anti-CD137 scFv is linked to a heavy chain carboxy terminal of the anti-CLDN4 IgG antibody via a linker.
[32] An anti-CLDN4/anti-CD137 bispecific antibody for treating cancer of a subject, which is used in combination with a PD-1 signal inhibitor, wherein the bispecific antibody comprises a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region consisting of an amino acid sequence at amino acid positions 1-109 of SEQ ID NO: 4, and an anti-CD 137 scFv comprising a light chain variable region of an anti-CD 137 antibody consisting of an amino acid sequence at amino acid positions 464-573 of SEQ ID NO: 2 and a heavy chain variable region of an anti-CD137 antibody consisting of an amino acid sequence at amino acid positions 595-712 of SEQ ID NO: 2, wherein an amino terminal of the anti-CD137 scFv is linked to a heavy chain carboxy terminal of the anti-CLDN4 antibody via a linker.
[33] An anti-CLDN4/anti-CD137 bispecific antibody for treating cancer of a subject, which is used in combination with a PD-1 signal inhibitor, wherein the bispecific antibody comprises a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4, and an anti-CD137 scFv consisting of an amino acid sequence at amino acid positions 464-712 of SEQ ID NO: 2, wherein an amino terminal of the anti-CD137 scFv is linked to a heavy chain carboxy terminal of the anti-CLDN4 antibody via a linker.
[34] The bispecific antibody according to any of [31] to [33], wherein the linker is a GS linker.
[35] An anti-CLDN4/anti-CD137 bispecific antibody used for treating cancer of a subject, comprising a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4, wherein the bispecific antibody is used in combination with a PD-1 signal inhibitor.
[36] The anti-CLDN4/anti-CD137 bispecific antibody according to any of [22] to [35], wherein the anti-CLDN4/anti-CD137 bispecific antibody is post-translationally modified.
[37] The bispecific antibody according to any of [22] to [36], which is used in combination with the PD-1 signal inhibitor simultaneously, continuously, or sequentially.
[38] The bispecific antibody according to any of [22] to [37], wherein the anti-CLDN4/anti-CD137 bispecific antibody and the PD-1 signal inhibitor are (i) comprised in the same pharmaceutical composition and administered simultaneously or (ii) comprised in separate pharmaceutical compositions and used in combination simultaneously, continuously, or sequentially.
[39] The bispecific antibody according to any of [22] to [38], wherein the cancer is selected from the group consisting of colorectal cancer, bladder cancer, and lung cancer.
[40] The bispecific antibody according to any of [22] to [39], wherein the PD-1 signal inhibitor is an antibody binding to one or more proteins selected from the group consisting of PD-1, PD-L1, and PD-L2, or an antigen binding fragment thereof.
[41] The bispecific antibody according to any of [22] to [40], wherein the PD-1 signal inhibitor is an anti-PD-1 antibody selected from the group consisting of nivolumab, pembrolizumab, pidilizumab, spartalizumab, and cemiplimab.
[42] The bispecific antibody according to any of [22] to [40], wherein the PD-1 signal inhibitor is an anti-PD-L1 antibody selected from the group consisting of atezolizumab, durvalumab, and avelumab.
[43] A method for treating cancer, comprising administering an anti-CLDN4/anti-CD137 bispecific antibody in combination with a PD-1 signal inhibitor to a subject, wherein the bispecific antibody comprises a heavy chain variable region and a light chain variable region of an anti-CLDN4 antibody, and a heavy chain variable region and a light chain variable region of an anti-CD137 antibody.
[44] The method for treating cancer according to [43], wherein the heavy chain variable region of the anti-CLDN4 antibody comprises a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and the light chain variable region of the anti-CLDN4 antibody comprises a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4.
[45] The method for treating cancer according to [43] or [44], wherein the heavy chain variable region of the anti-CLDN4 antibody consists of an amino acid sequence at amino acid positions 1-123 of SEQ ID NO: 2, and the light chain variable region of the anti-CLDN4 antibody consists of an amino acid sequence at amino acid positions 1-109 of SEQ ID NO: 4.
[46] The method for treating cancer according to any of [43] to [45], wherein the anti-CLDN4/anti-CD137 bispecific antibody comprises an IgG antibody (anti-CLDN4 IgG antibody) consisting of a heavy chain comprising the heavy chain variable region of the anti-CLDN4 antibody and a light chain comprising the light chain variable region of the anti-CLDN4 antibody.
[47] The method for treating cancer according to [46], comprising either LALA mutation (L234A and L235A) or P331G mutation (wherein a position of the mutation is an amino acid position in human Igγ1 constant region according to EU index) or both in a Fc region of the anti-CLDN4 IgG antibody.
[48] The method for treating cancer according to any of [43] to [47], wherein the heavy chain variable region of the anti-CD137 antibody comprises a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and the light chain variable region of the anti-CD 137 antibody comprises a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2.
[49] The method for treating cancer according to any of [43] to [48], wherein the heavy chain variable region of the anti-CD137 antibody consists of an amino acid sequence at amino acid positions 595-712 of SEQ ID NO: 2, and the light chain variable region of the anti-CD137 antibody consists of an amino acid sequence at amino acid positions 464-573 of SEQ ID NO: 2.
[50] The method for treating cancer according to [48] or [49], wherein the anti-CLDN4/anti-CD137 bispecific antibody comprises an anti-CD137 single chain variable fragment (anti-CD137 scFv) comprising the heavy chain variable region and the light chain variable region of the anti-CD137 antibody.
[51] The method for treating cancer according to [50], wherein the anti-CD137 scFv consists of an amino acid sequence at amino acid positions 464-712 of SEQ ID NO: 2.
[52] The method for treating cancer according to [50] or [51], wherein the anti-CLDN4/anti-CD137 bispecific antibody comprises an anti-CLDN4 IgG antibody and the anti-CD137 scFv, and an amino terminal of the anti-CD 137 scFv is linked to a heavy chain carboxy terminal of the anti-CLDN4 IgG antibody via a linker.
[53] A method for treating cancer, comprising administering an anti-CLDN4/anti-CD137 bispecific antibody in combination with a PD-1 signal inhibitor to a subject, wherein the bispecific antibody comprises a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region consisting of an amino acid sequence at amino acid positions 1-109 of SEQ ID NO: 4, and an anti-CD137 scFv comprising a light chain variable region of an anti-CD137 antibody consisting of an amino acid sequence at amino acid positions 464-573 of SEQ ID NO: 2 and a heavy chain variable region of an anti-CD 137 antibody consisting of an amino acid sequence at amino acid positions 595-712 of SEQ ID NO: 2, wherein an amino terminal of the anti-CD137 scFv is linked to a heavy chain carboxy terminal of the anti-CLDN4 antibody via a linker.
[54] A method for treating cancer, comprising administering an anti-CLDN4/anti-CD137 bispecific antibody in combination with a PD-1 signal inhibitor to a subject, wherein the bispecific antibody comprises a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4, and an anti-CD137 scFv consisting of an amino acid sequence at amino acid positions 464-712 of SEQ ID NO: 2, wherein an amino terminal of the anti-CD137 scFv is linked to a heavy chain carboxy terminal of the anti-CLDN4 antibody via a linker.
[55] The method for treating cancer according to any of [52] to [54], wherein the linker is a GS linker.
[56] A method for treating cancer, comprising administering an anti-CLDN4/anti-CD137 bispecific antibody in combination with a PD-1 signal inhibitor to a subject, wherein the bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4.
[57] The method for treating cancer according to any one of [43] to [56], wherein the anti-CLDN4/anti-CD137 bispecific antibody is post-translationally modified.
[58] The method for treating cancer according to any of [43] to [57], wherein the anti-CLDN4/anti-CD137 bispecific antibody is used in combination with the PD-1 signal inhibitor simultaneously, continuously, or sequentially.
[59] The method for treating cancer according to any of [43] to [58], wherein the anti-CLDN4/anti-CD137 bispecific antibody and the PD-1 signal inhibitor are (i) comprised in the same pharmaceutical composition and administered simultaneously or (ii) comprised in separate pharmaceutical compositions and used in combination simultaneously, continuously, or sequentially.
[60] The method for treating cancer according to any of [43] to [58], wherein the cancer is selected from the group consisting of colorectal cancer, bladder cancer, and lung cancer.
[61] The method for treating cancer according to any one of [43] to [60], wherein the PD-1 signal inhibitor is an antibody binding to one or more proteins selected from the group consisting of PD-1, PD-L1, and PD-L2, or an antigen binding fragment thereof.
[62] The method for treating cancer according to any of [43] to [61], wherein the PD-1 signal inhibitor is an anti-PD-1 antibody selected from the group consisting of nivolumab, pembrolizumab, pidilizumab, spartalizumab, and cemiplimab.
[63] The method for treating cancer according to any of [43] to [61], wherein the PD-1 signal inhibitor is an anti-PD-L1 antibody selected from the group consisting of atezolizumab, durvalumab, and avelumab.
[64] Use of an anti-CLDN4/anti-CD137 bispecific antibody for production of a pharmaceutical composition used in combination with a PD-1 signal inhibitor to treat cancer of a subject, wherein the bispecific antibody comprises a heavy chain variable region and a light chain variable region of an anti-CLDN4 antibody, and a heavy chain variable region and a light chain variable region of an anti-CD 137 antibody.
[65] The use according to [64], wherein the heavy chain variable region of the anti-CLDN4 antibody comprises a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and the light chain variable region of the anti-CLDN4 antibody comprises a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4.
[66] The use according to [64] or [65], wherein the heavy chain variable region of the anti-CLDN4 antibody consists of an amino acid sequence at amino acid positions 1-123 of SEQ ID NO: 2, and the light chain variable region of the anti-CLDN4 antibody consists of an amino acid sequence at amino acid positions 1-109 of SEQ ID NO: 4.
[67] The use according to any of [64] to [66], wherein the anti-CLDN4/anti-CD137 bispecific antibody comprises an IgG antibody (anti-CLDN4 IgG antibody) consisting of a heavy chain comprising the heavy chain variable region of the anti-CLDN4 antibody and a light chain comprising the light chain variable region of the anti-CLDN4 antibody.
[68] The use according to [67], comprising either LALA mutation (L234A and L235A) or P331G mutation (wherein a position of the mutation is an amino acid position in human Igγ1 constant region according to EU index) or both in a Fc region of the anti-CLDN4 IgG antibody.
[69] The use according to any of [64] to [68], wherein the heavy chain variable region of the anti-CD137 antibody comprises a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and the light chain variable region of the anti-CD137 antibody comprises a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2.
[70] The use according to any of [64] to [69], wherein the heavy chain variable region of the anti-CD137 antibody consists of an amino acid sequence at amino acid positions 595-712 of SEQ ID NO: 2, and the light chain variable region of the anti-CD137 antibody consists of an amino acid sequence at amino acid positions 464-573 of SEQ ID NO: 2.
[71] The use according to [69] or [70], wherein the anti-CLDN4/anti-CD137 bispecific antibody comprises an anti-CD137 single chain variable fragment (anti-CD137 scFv) comprising the heavy chain variable region and the light chain variable region of the anti-CD137 antibody.
[72] The use according to [71], wherein the anti-CD137 scFv consists of an amino acid sequence at amino acid positions 464-712 of SEQ ID NO: 2.
[73] The use according to [71] or [72], wherein the anti-CLDN4/anti-CD137 bispecific antibody comprises an anti-CLDN4 IgG antibody and the anti-CD137 scFv, and an amino terminal of the anti-CD137 scFv is linked to a heavy chain carboxy terminal of the anti-CLDN4 IgG antibody via a linker.
[74] Use of an anti-CLDN4/anti-CD137 bispecific antibody for production of a pharmaceutical composition used in combination with a PD-1 signal inhibitor to treat cancer of a subject, wherein the bispecific antibody comprises a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region consisting of an amino acid sequence at amino acid positions 1-109 of SEQ ID NO: 4, and an anti-CD137 scFv comprising a light chain variable region of an anti-CD137 antibody consisting of an amino acid sequence at amino acid positions 464-573 of SEQ ID NO: 2 and a heavy chain variable region of an anti-CD137 antibody consisting of an amino acid sequence at amino acid positions 595-712 of SEQ ID NO: 2, wherein an amino terminal of the anti-CD137 scFv is linked to a heavy chain carboxy terminal of the anti-CLDN4 antibody via a linker.
[75] Use of an anti-CLDN4/anti-CD137 bispecific antibody for production of a pharmaceutical composition used in combination with a PD-1 signal inhibitor to treat cancer of a subject, wherein the bispecific antibody comprises a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4, and an anti-CD137 scFv consisting of an amino acid sequence at amino acid positions 464-712 of SEQ ID NO: 2, wherein an amino terminal of the anti-CD137 scFv is linked to a heavy chain carboxy terminal of the anti-CLDN4 antibody via a linker.
[76] The use according to any of [73] to [75], wherein the linker is a GS linker.
[77] Use of an anti-CLDN4/anti-CD137 bispecific antibody for production of a pharmaceutical composition used in combination with a PD-1 signal inhibitor to treat cancer of a subject, wherein the bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4.
[78] The use according to any of [64] to [77], wherein the anti-CLDN4/anti-CD137 bispecific antibody is post-translationally modified.
[79] The use according to any one of [64] to [78], wherein the pharmaceutical composition is used in combination with the PD-1 signal inhibitor simultaneously, continuously, or sequentially.
[80] The use according to any of [64] to [79], wherein the anti-CLDN4/anti-CD137 bispecific antibody and the PD-1 signal inhibitor are (i) comprised in the same pharmaceutical composition and administered simultaneously or (ii) comprised in separate pharmaceutical compositions and used in combination simultaneously, continuously, or sequentially.
[81] The use according to any of [64] to [80], wherein the cancer is selected from the group consisting of colorectal cancer, bladder cancer, and lung cancer.
[82] The use according to any of [64] to [81], wherein the PD-1 signal inhibitor is an antibody binding to one or more proteins selected from the group consisting of PD-1, PD-L1, and PD-L2, or an antigen binding fragment thereof.
[83] The use according to any of [64] to [82], wherein the PD-1 signal inhibitor is an anti-PD-1 antibody selected from the group consisting of nivolumab, pembrolizumab, pidilizumab, spartalizumab, and cemiplimab.
[84] The use according to any of [64] to [82], wherein the PD-1 signal inhibitor is an anti-PD-1 antibody selected from the group consisting of atezolizumab, durvalumab, and avelumab.

### ADVANTAGEOUS EFFECTS OF INVENTION

An anti-CLDN4/anti-CD137 bispecific antibody of the present invention binds both to CLDN4 highly expressing in cancer and CD137 that is a T cell surface molecule to activate immune cells present around a cancer cell, and thus enhances killing activity on the cancer cell. The combination of the anti-CLDN4/anti-CD137 bispecific antibody of the present invention and a PD-1 signal inhibitor results in a significant antitumor effect compared with administration of either the anti-CLDN4/anti-CD137 bispecific antibody or the PD-1 signal inhibitor alone. Therefore, the present invention provides use of the anti-CLDN4/anti-CD137 bispecific antibody in combination with the PD-1 signal inhibitor in cancer treatment.

### BRIEF DESCRIPTION OF DRAWINGS

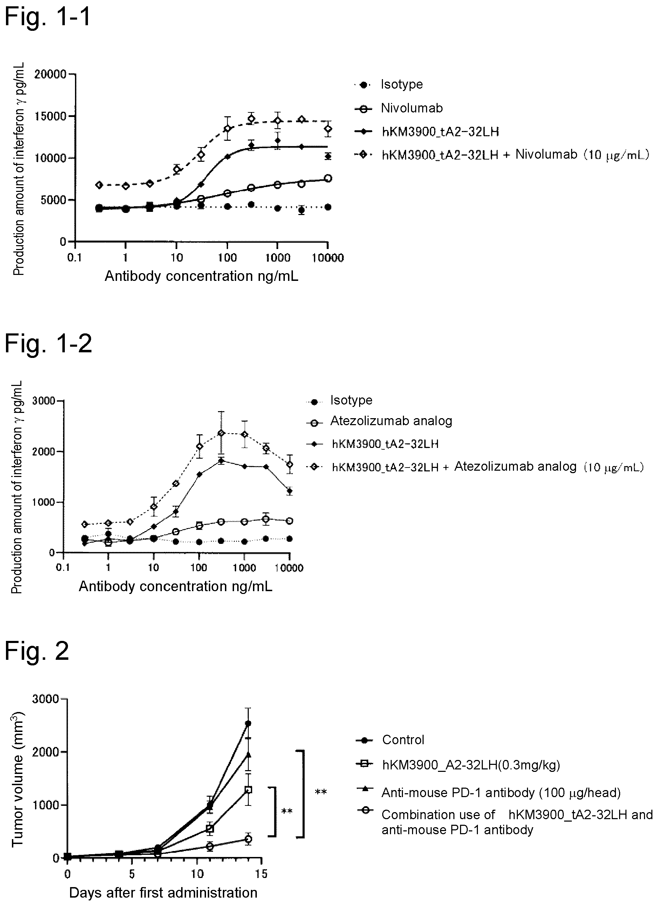

[Fig. 1-1] Fig. 1-1 illustrates a production amount of interferon y obtained by adding a test antibody to a coculture system of LCLC-OKT3 scFv cells of a human large cell lung cancer cell line and expanded pan T cells. The ordinate of the drawing indicates a production amount of interferon y obtained 4 days after the antibody addition, and the abscissa indicates a concentration of the antibody. Each of symbols indicates an average of the production amount of interferon y at each concentration of the antibody. An error bar indicates the standard deviation.
[Fig. 1-2] Fig. 1-2 illustrates a production amount of interferon y obtained by adding test antibodies to a coculture system of LCLC-OKT3 scFv cells of a human large cell lung cancer cell line and expanded pan T cells. The ordinate of the drawing indicates a production amount of interferon y obtained 5 days after the antibody addition, and the abscissa indicates a concentration of the antibody. Each of symbols indicates an average of the production amount of interferon y at each concentration of the antibody. An error bar indicates the standard deviation.
[Fig. 2] Fig. 2 illustrates an inhibitory effect on the growth of human CLDN4 expressing B16-F10 cells which are tumor-bearing on B-h4-1BB mice. Fig. 2 shows an average tumor volume (n = 10) on each day after the start of antibody administration. An error bar indicates the standard error of the tumor volume. The ordinate of the drawing indicates the tumor volume, and the abscissa indicates days after the first antibody administration. Significance probability P-values were obtained by comparing the tumor volume in the combination use group and the test antibody alone administration group through unpaired Student's t-test. In the drawing, ** indicates a p-value smaller than a significance level of 0.01.

### DESCRIPTION OF EMBODIMENTS

The present invention will now be described in detail.

Terms used herein are used to have meanings generally used in this technical field by those skilled in the art unless especially defined in the following.

An antibody (or immunoglobulin) refers to a glycoprotein whose basic structure is a four-chain structure of a bilaterally symmetrical Y-shape including two heavy chains having a single sequence and two light chains having a single sequence. There are five classes of antibody, that is, IgG, IgM, IgA, IgD and IgE. A basic structure of an antibody molecule is common among these classes, and two heavy chains having a molecular weight of 50,000 to 70,000 and two light chains having a molecular weight of 20,000 to 30,000 are bound via a disulfide bond and a noncovalent bond to form an antibody molecule having a Y-shaped four chain structure with a molecular weight of 150,000 to 190,000. The heavy chain is a polypeptide chain usually comprising about 440 amino acids, and has a structure peculiar to each class, and is designated respectively as Igγ, Igµ, Igα, Igδ, and Igε correspondingly to IgG, IgM, IgA, IgD, or IgE. IgG has subclasses of IgG1, IgG2, IgG3 and IgG4, and heavy chains respectively corresponding to these subclasses are designated as Igy1, Igy2, Igy3, and Igy4. The light chain is a polypeptide chain usually containing about 220 amino acids, and it is known to have two types, λ type and κ type, which are respectively designated as Igλ and Igκ. Each of the two types of the light chains can be paired with any type of the heavy chain.

In an antibody molecule, the heavy chain has four intrachain disulfide bonds (five in Igµ and Igε), the light chain has two intrachain disulfide bonds, and thus, one loop is formed every 100 to 110 amino acid residues. The three-dimensional structures of these are similar among the loops, and are designated as a structural unit or a domain. In both the heavy chain and the light chain, a domain positioned at the amino terminal (herein also referred to as the "N-terminal") is designated as a variable region, and has an amino acid sequence varied even among antibodies produced from the same class (or subclass) of the same species of animal, and is known to be involved in antibody-antigen bond specific binding. A domain disposed on the downstream side from the variable region on the C-terminal side has an amino acid sequence substantially constant in each class or subclass, and is designated as a constant region. The heavy chain has, in a direction from the N-terminal to the carboxy terminal (herein also referred to as the "C-terminal"), a heavy chain variable region (VH) and a heavy chain constant region (CH). The CH is further divided, from the N-terminal side, into three domains of a CH1 domain, a CH2 domain and a CH3 domain. The light chain has, in a direction from the N-terminal to the C-terminal, a light chain variable region (VL) and a light chain constant region (CL).

The amino acid sequence of three complementarity determining regions (CDRs) present in each of the VH and VL are highly variable, contributing to variability of the variable regions. Each CDR is a region consisting of about 5 to 10 amino acid residues, which is present in the order of CDR1, CDR2 and CDR3 in the N-terminal of each of the heavy chain and the light chain, and forms an antigen binding site. On the other hand, a portion excluding the CDRs in the variable region is designated as a frame work region (FR), which consists of FR1 to FR4, with relatively little variation in the amino acid sequence.

When an antibody is treated with papain, that is, a protease, three antibody fragments are obtained. Two fragments disposed on the N-terminal side are designated as a Fab (antigen binding fragment, Fragment, antigen binding) region. Herein, a "Fab region" refers to a region consisting of a VH and a CH1 domain of a heavy chain and a light chain (VL and CL), and binds to an antigen at a tip portion of the antigen binding site included in the Fab region. Herein, a "heavy chain fragment" refers to a fragment consisting of a VH and a CH1 domain of a heavy chain included in the Fab region. Besides, a fragment disposed on the C-terminal side is designated as a "Fc (crystallizable fragment, Fragment, crystallizable) region."

Herein, the term "antigen" is used in a generally used sense, especially as a term for a molecule or a part of a molecule to which an antigen binding protein such as an antibody or an antigen binding fragment can specifically bind. The antigen may be a molecule such as a protein or a nucleic acid. One antigen may have one or more epitopes capable of interacting with different antibodies and the like.

Herein, an "IgG antibody" refers to an antibody having a Y-shaped structure including two Fab regions and a Fc region. In one embodiment, the two Fab regions of the IgG antibody contain the same Fab region sequences.

Herein, an "antigen binding fragment" is a molecule that comprises at least one polypeptide chain having antigen binding activity derived from an antibody. Representative examples of the antigen binding fragment include a single chain variable fragment (scFv), a Fab fragment, a Fab' fragment, and a F(ab')₂ fragment. The scFv is a monovalent antigen binding fragment comprising a VH and a VL linked via a linker. A Fab fragment is a monovalent antigen binding fragment comprising a fragment consisting of a light chain, and a VH and a CH1 domain of a heavy chain. A Fab' fragment is a monovalent antigen binding fragment comprising a fragment consisting of a light chain, a VH and a CH1 domain of a heavy chain, and a part of a hinge region, and this part of the hinge region comprises a cysteine residue comprised in the S-S bond between the heavy chains. A F(ab')₂ fragment is a bivalent molecule comprising Fab' fragments bounded via a disulfide bond. The term "monovalent" means that one antigen binding site is comprised, and the term "bivalent" means that two antigen binding sites are comprised.

Herein, a "bispecific antibody" refers to an antibody capable of specifically binding to two different antigens. The term "anti-CLDN4/anti-CD137 bispecific antibody" means a bispecific antibody having binding activity to CLDN4 and binding activity to CD137.

Herein, the term "antibody" is used to encompass a full length antibody, an antigen binding fragment, and a bispecific antibody having any structure unless particularly limited contextually.

Herein, a "human antibody" refers to an antibody having a human immunoglobulin amino acid sequence. Herein, a "humanized antibody" refers to an antibody in which a part of, most of, or all of amino acid residues excluding the CDRs are substituted with amino acid residues derived from a human immunoglobulin molecule. A humanization method is not especially limited, and for example, U.S. Patent No. 5225539, U.S. Patent No. 6180370 and the like can be referred to for producing a humanized antibody.

An amino acid residue number of an antibody used herein can be prescribed by specifying the Kabat numbering or EU index (Kabat et al., Sequences of Proteins of Immunological Interest, 5th Ed., 1991: NIH Publication: No. 91-3242) in accordance with the numbering system.

Herein, the term "link," "conjugate," or "linked" means that a plurality of components (such as an IgG antibody and a scFv) are connected directly or via an intermediary (such as a peptide linker). Herein, the term "peptide linker" means at least one amino acid sequence that can be introduced by a genetic engineering method used for linking a plurality of components. The length of a peptide linker used in the present invention is not especially limited, and can be appropriately selected by those skilled in the art depending on purpose.

Herein, the term "subject" means a human or other animals in need of prevention or treatment of a disease. In one embodiment, the subject is a human in need of prevention or treatment of a disease. In one embodiment, the subject is a human having cancer.

Herein, the term "treatment" means any therapeutic intervention, procedure, or administration of an active ingredient to a subject that is performed on the subject for the purpose of reversing, alleviating, ameliorating, inhibiting, or delaying the progression, onset, severity, or recurrence of a disease symptom, condition, or disease-associated biochemical sign.

Herein, the term "active ingredient" means a substance that exhibits some physiological activity among those comprised in a pharmaceutical composition, a pharmaceutical, or the like used for the prevention or treatment of a disease. In one embodiment, the active ingredient is an antibody, a low molecular weight compound, a nucleic acid, a fusion protein, or a peptide. In one embodiment, the active ingredient is an antibody. In one embodiment, the active ingredient is a bispecific antibody.

Herein, a "pharmaceutical composition" is a drug comprising an active ingredient and a pharmaceutically acceptable excipient (for example, including, but not limited to, a pharmaceutical excipient, a pharmaceutical carrier, or the like) that is formulated for treatment of a subject.

Herein, the term "combination use," "combination," or "used in combination" means administration of multiple active ingredients simultaneously, continuously, or sequentially to the same subject for prevention or treatment of a disease. The multiple active ingredients may be comprised in the same pharmaceutical composition or may be comprised separately in different pharmaceutical compositions. Herein, "simultaneously" means administration of multiple active ingredients in parallel within one administration period; "continuously" means administration of one active ingredient after the completion of the administration of the other active ingredient without interval; and "sequentially" means administration of multiple active ingredients in sequence according to an administration schedule.

Herein, an "effective amount" of a drug refers to the amount of the drug required to effect a physiological change in a cell or tissue to which it is administered.

Herein, a "PD-1 signal inhibitor" means a drug that removes suppression of immune cell activation by PD-1. The PD-1 signal inhibitor binds to a PD-1 or its ligand PD-L1 or PD-L2 to inhibit an immunosuppressive signal, and thus can inhibit the immune checkpoint function of PD-1. The PD-1 signal inhibitor may be any substance as long as it has an effect of blocking a PD-1 signal, for example, an antibody, a low molecular weight compound, a nucleic acid (which may include DNA or RNA, or a natural or artificial nucleic acid), a fusion protein, or a peptide. For example, an anti-PD-1 antibody, an anti-PD-L1 antibody, or an anti-PD-L2 antibody can inhibit a PD-1 signal by inhibiting the binding between PD-1 and PD-L1 or PD-L2 (Expert Opinion on Therapeutic Patents, 2016: Vol. 26: p. 555-564).

The present invention relates to the following (1) to (4):
(1) a pharmaceutical composition used in combination with a PD-1 signal inhibitor, comprising an anti-CLDN4/anti-CD137 bispecific antibody (herein also referred to as the "pharmaceutical composition of the present invention");
(2) an anti-CLDN4/anti-CD137 bispecific antibody used in combination with a PD-1 signal inhibitor to treat cancer of a subject;
(3) a method for treating cancer, including administering an anti-CLDN4/anti-CD137 bispecific antibody and a PD-1 signal inhibitor to a subject (herein also referred to as the "therapeutic method of the present invention"); or
(4) use of an anti-CLDN4/anti-CD137 bispecific antibody for production of a pharmaceutical composition used in combination with a PD-1 signal inhibitor to treat cancer of a subject.

### <Anti-CLDN4/anti-CD137 Bispecific Antibody of Invention>

A bispecific antibody binding to CLDN4 and CD137 used in the present invention (also referred to as the "anti-CLDN4/anti-CD137 bispecific antibody of the present invention") comprises a heavy chain variable region and a light chain variable region of an anti-CLDN4 antibody, and a heavy chain variable region and a light chain variable region of an anti-CD137 antibody.

Herein, the "anti-CLDN4 antibody" is an antibody capable of binding to human CLDN4, and the "anti-CD137 antibody" is an antibody capable of binding to human CD137. It can be checked by employing a known binding activity measurement method whether or not an antibody binds to human CLDN4 or human CD137. Examples of a method for measuring binding activity include a method such as enzyme-linked immunosorbent assay (ELISA) or flow cytometry. ELISA or flow cytometry can be carried out by a method usually employed by those skilled in the art.

The anti-CLDN4/anti-CD137 bispecific antibody of the present invention may have any structure as long as it binds to CLDN4 and CD137, and examples thereof include a bispecific antibody having a structure described in NPL 6. In one embodiment, the anti-CLDN4/anti-CD137 bispecific antibody of the present invention may be a conjugate in which a Fab region of an anti-CLDN4 antibody and a Fab region of an anti-CD 137 antibody are linked to each other, a conjugate of an anti-CLDN4 antibody of the IgG antibody type (also referred to as the "anti-CLDN4 IgG antibody") and an anti-CD 137 antibody of the IgG antibody type (also referred to as the "anti-CD137 IgG antibody"), a conjugate of an anti-CLDN4 IgG antibody and an antigen binding fragment of an anti-CD137 antibody, a conjugate of an antigen binding fragment of an anti-CLDN4 antibody and an anti-CD137 IgG antibody, or a conjugate of an antigen binding fragment of an anti-CLDN4 antibody and an antigen binding fragment of an anti-CD 137 antibody.

In one embodiment, the anti-CLDN4/anti-CD137 bispecific antibody of the present invention comprises a heavy chain variable region of an anti-CLDN4 antibody comprising a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and a light chain variable region of an anti-CLDN4 antibody comprising a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4.

In one embodiment, the anti-CLDN4/anti-CD137 bispecific antibody of the present invention comprises a heavy chain variable region of an anti-CLDN4 antibody consisting of an amino acid sequence at amino acid positions 1-123 of SEQ ID NO: 2 and a light chain variable region of an anti-CLDN4 antibody consisting of an amino acid sequence at amino acid positions 1-109 of SEQ ID NO: 4.

The anti-CLDN4 antibody comprised in the anti-CLDN4/anti-CD137 bispecific antibody of the present invention may be an IgG antibody. As the heavy chain constant region comprised in the anti-CLDN4 antibody, any of the constant regions Igγ, Igµ, Igα, Igδ and Igε can be selected. Igγ can be selected from, for example, Igγ1, Igy2, Igy3 and Igy4. As the light chain constant region comprised in the anti-CLDN4 antibody comprised in the anti-CLDN4/anti-CD137 bispecific antibody of the present invention, either of the constant regions Igλ and Igκ can be selected. The heavy chain and the light chain of the anti-CLDN4 antibody are respectively human Igγ1 and Igκ in one embodiment. In one embodiment, the anti-CLDN4/anti-CD137 bispecific antibody of the present invention comprises a full length anti-CLDN4 antibody. In one embodiment, the anti-CLDN4 antibody comprised in the anti-CLDN4/anti-CD137 bispecific antibody of the present invention is an IgG antibody comprising a heavy chain variable region and a light chain variable region of an anti-CLDN4 antibody (anti-CLDN4 IgG antibody).

When the anti-CLDN4/anti-CD137 bispecific antibody of the present invention comprises a Fc region, the Fc region of the bispecific antibody may contain mutation that deteriorates antibody-dependent cellular cytotoxicity (ADCC) or complement-dependent cytotoxicity (CDC). L234A is replacement of leucine with alanine at amino acid position 234 in human Igγ1 constant region. L235A is replacement of leucine with alanine at amino acid position 235 in human Igγ1 constant region. The amino acid mutation L234A and L235A of human Igγ1 constant region is designated as "LALA mutation." This mutation is known to deteriorate ADCC and CDC of an antibody (Mol. Immunol., 1992, Vol. 29: p. 633-639, and J. Immunol., 2000: Vol. 164(8): p. 4178-4184). P331G or P331S is replacement of proline with glycine or serine at amino acid position 331 in human Igγ1 constant region. This mutation is known to deteriorate CDC of an antibody (J. Immunol., 2000: Vol. 164(8): p. 4178-4184).

In one embodiment, the anti-CLDN4 IgG antibody comprised in the anti-CLDN4/anti-CD137 bispecific antibody of the present invention comprises a Fc region comprising amino acid mutation L234A and L235A (LALA mutation). In one embodiment, the anti-CLDN4 IgG antibody comprises a Fc region comprising either P331G or P331S mutation. In one embodiment, the anti-CLDN4 IgG antibody comprises a Fc region comprising mutations of LALA mutation and either P331G or P331S mutation. In one embodiment, the anti-CLDN4 IgG antibody comprises a Fc region comprising either LALA mutation or P331G mutation or both mutations.

Note that the description of an amino acid mutation such as LALA mutation or P331G or P331S mutation herein is based on an amino acid position in human Igγ1 constant region according to EU index. For example, as described above, L234A is a replacement of leucine with alanine at amino acid position 234 in human Igγ1 constant region according to EU index.

In one embodiment, the anti-CLDN4/anti-CD137 bispecific antibody of the present invention is an IgG antibody comprising a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4.

In one embodiment, the heavy chain variable region of the anti-CD 137 antibody comprised in the anti-CLDN4/anti-CD137 bispecific antibody of the present invention comprises a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and the light chain variable region of the anti-CD137 antibody comprises a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2.

In one embodiment, the heavy chain variable region of the anti-CD 137 antibody comprised in the anti-CLDN4/anti-CD137 bispecific antibody consists of an amino acid sequence at amino acid positions 595-712 of SEQ ID NO: 2, and the light chain variable region of the anti-CD137 antibody consists of an amino acid sequence at amino acid positions 464-573 of SEQ ID NO: 2.

In one embodiment, the anti-CLDN4/anti-CD137 bispecific antibody of the present invention comprises a scFv of an anti-CD 137 antibody (herein referred to as the "anti-CD137 scFv").

The type and the length of a linker used for linking the heavy chain variable region and the light chain variable region of the anti-CD137 antibody in the anti-CD137 scFv are not especially limited, and can be appropriately selected by those skilled in the art. As the linker, a peptide linker may be used. A preferable length is 5 or more amino acids (with an upper limit being not especially limited, but being usually 30 or less amino acids, and preferably 20 or less amino acids), and is particularly preferably 15 amino acids. As the linker, for example, a glycine-serine linker (GS linker), or a glycine-lysine-proline-glycine-serine linker (GKPGS linker) can be used. Examples of linkers in the present invention include the following:
Ser
Gly-Ser
Gly-Gly-Ser
Ser-Gly-Gly
Gly-Gly-Gly-Ser (SEQ ID No. 5)
Ser-Gly-Gly-Gly (SEQ ID No. 6)
Gly-Gly-Gly-Gly-Ser (SEQ ID No. 7)
Ser-Gly-Gly-Gly-Gly (SEQ ID No. 8)
Gly-Gly-Gly-Gly-Gly-Ser (SEQ ID No. 9)
Ser-Gly-Gly-Gly-Gly-Gly (SEQ ID No. 10)
Gly-Gly-Gly-Gly-Gly-Gly-Ser (SEQ ID No. 11)
Ser-Gly-Gly-Gly-Gly-Gly-Gly (SEQ ID No. 12)
Gly-Gly-Gly-Gly-Ser-Gly-Gly-Gly-Gly-Ser (SEQ ID No. 13)
(Gly-Gly-Gly-Gly-Ser)n
(Ser-Gly-Gly-Gly-Gly)n
Gly-Lys-Pro-Gly-Ser (SEQ ID No. 14)
(Gly-Lys-Pro-Gly-Ser)n

In the above-described examples, n represents an integer of 1 or more. In one aspect, n is 1 to 10, 2 to 8, or 2 to 6. The length and the sequence of the linker can be appropriately selected depending on purpose by those skilled in the art.

In one embodiment, the linker used in the anti-CD137 scFv is a GS linker of (Gly-Gly-Gly-Gly-Ser)n.

In one embodiment, the linker used in the anti-CD137 scFv is a GS linker of (Gly-Gly-Gly-Gly-Ser)4.

In one embodiment, the anti-CLDN4/anti-CD 137 bispecific antibody of the present invention comprises an anti-CD137 scFv in which a light chain variable region consisting of an amino acid sequence at amino acid positions 464-573 of SEQ ID NO: 2 is linked to a heavy chain variable region consisting of an amino acid sequence at amino acid positions 595-712 of SEQ ID NO: 2 via a GS linker.

In one embodiment, the anti-CLDN4/anti-CD 137 bispecific antibody of the present invention comprises an anti-CD137 scFv consisting of an amino acid sequence at amino acid positions 464-712 of SEQ ID NO: 2.

In one embodiment, the anti-CLDN4/anti-CD 137 bispecific antibody of the present invention comprises an anti-CLDN4 IgG antibody and an anti-CD137 scFv.

In the anti-CLDN4/anti-CD 137 bispecific antibody of the present invention, the anti-CLDN4 antibody or its antigen binding fragment may be linked to the anti-CD137 antibody or its antigen binding fragment (such as an anti-CD137 scFv) via a linker. In one embodiment, the anti-CLDN4/anti-CD137 bispecific antibody of the present invention comprises an anti-CLDN4 IgG antibody and an anti-CD137 scFv, in which the anti-CLDN4 IgG antibody is linked to the anti-CD137 scFv via a linker. In one embodiment, the anti-CLDN4/anti-CD137 bispecific antibody of the present invention comprises an anti-CLDN4 IgG antibody and an anti-CD137 scFv, in which the amino terminal of the anti-CD137 scFv is linked to the heavy chain carboxy terminal of the anti-CLDN4 IgG antibody via a linker. The type and the length of the linker for linking the anti-CLDN4 antibody or its antigen binding fragment to the anti-CD 137 antibody or its antigen binding fragment are not especially limited, and can be appropriately selected by those skilled in the art. As the linker, a peptide linker may be used. A preferable length is 5 or more amino acids (with an upper limit being not especially limited, but being usually 30 or less amino acids, and preferably 20 or less amino acids), and is particularly preferably 10 amino acids. As the peptide linker, for example, a glycine-serine linker (GS linker), or a glycine-lysine-proline-glycine-serine linker (GKPGS linker) can be used. Examples of linkers in the present invention include the following:
Ser
Gly-Ser
Gly-Gly-Ser
Ser-Gly-Gly
Gly-Gly-Gly-Ser (SEQ ID No. 5)
Ser-Gly-Gly-Gly (SEQ ID No. 6)
Gly-Gly-Gly-Gly-Ser (SEQ ID No. 7)
Ser-Gly-Gly-Gly-Gly (SEQ ID No. 8)
Gly-Gly-Gly-Gly-Gly-Ser (SEQ ID No. 9)
Ser-Gly-Gly-Gly-Gly-Gly (SEQ ID No. 10)
Gly-Gly-Gly-Gly-Gly-Gly-Ser (SEQ ID No. 11)
Ser-Gly-Gly-Gly-Gly-Gly-Gly (SEQ ID No. 12)
Gly-Gly-Gly-Gly-Ser-Gly-Gly-Gly-Gly-Ser (SEQ ID No. 13)
(Gly-Gly-Gly-Gly-Ser)n
(Ser-Gly-Gly-Gly-Gly)n
Gly-Lys-Pro-Gly-Ser (SEQ ID No. 14)
(Gly-Lys-Pro-Gly-Ser)n

In the above-described examples, n represents an integer of 1 or more. In one aspect, n is 1 to 10, 2 to 8, or 2 to 6. The length and the sequence of the peptide linker can be appropriately selected depending on purpose by those skilled in the art.

In one embodiment, a linker used as the peptide linker for linking the anti-CLDN4 antibody or its antigen binding fragment to the anti-CD 137 antibody or its antigen binding fragment (such as an anti-CD137 scFv) is a linker having an amino acid sequence of SEQ ID NO: 13.

In one embodiment, the anti-CLDN4/anti-CD137 bispecific antibody of the present invention is a bispecific antibody comprising: a heavy chain of an anti-CLDN4 antibody comprising a heavy chain variable region comprising a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and a light chain of an anti-CLDN4 antibody comprising a light chain variable region comprising a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4; and an anti-CD137 scFv comprising a heavy chain variable region of an anti-CD137 antibody comprising a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and a light chain variable region of an anti-CD137 antibody comprising a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2, in which the amino terminal of the anti-CD137 scFv is linked to the heavy chain carboxy terminal of the anti-CLDN4 antibody via a linker.

In one embodiment, the anti-CLDN4/anti-CD137 bispecific antibody of the present invention is a bispecific antibody comprising a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region of the anti-CLDN4 antibody consisting of an amino acid sequence at amino acid positions 1-109 of SEQ ID NO: 4, and an anti-CD137 scFv comprising a light chain variable region of an anti-CD137 antibody consisting of an amino acid sequence at amino acid positions 464-573 of SEQ ID NO: 2 and a heavy chain variable region of an anti-CD137 antibody consisting of an amino acid sequence at amino acid positions 595-712 of SEQ ID NO: 2, wherein an amino terminal of the anti-CD 137 scFv is linked to a heavy chain carboxy terminal of the anti-CLDN4 antibody via a linker.

In one embodiment, the anti-CLDN4/anti-CD 137 bispecific antibody of the present invention is a bispecific antibody comprising a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4, and an anti-CD137 scFv consisting of an amino acid sequence at amino acid positions 464-712 of SEQ ID NO: 2, wherein an amino terminal of the anti-CD137 scFv is linked to a heavy chain carboxy terminal of the anti-CLDN4 antibody via a linker.

In one embodiment, the anti-CLDN4/anti-CD 137 bispecific antibody of the present invention is a bispecific antibody comprising a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO 4.

Herein, the term "post-translational modification" refers to that an antibody expressed in a cell is modified after translation. Examples of the post-translational modification include modification such as pyroglutamylation, glycosylation, oxidation, deamidation or glycation of glutamine or glutamic acid at the heavy chain N-terminal, and lysine deletion by cutting lysine at the heavy chain C-terminal with carboxypeptidase. It is known that such post-translational modification is caused in various antibodies (J. Pharm. Sci., 2008: Vol. 97: p. 2426-2447).

In one embodiment, the anti-CLDN4/anti-CD 137 bispecific antibody of the present invention may be post-translationally modified. In one embodiment, the post-translational modification is pyroglutamylation at the N-terminal of the heavy chain variable region and/or lysine deletion at the heavy chain C-terminal. It is known in this field that the post-translational modification by pyroglutamylation at the N-terminal or lysine deletion at the C-terminal does not affect the activity of the antibody (Analytical Biochemistry, 2006, Vol. 348: p. 24-39).

The anti-CLDN4/anti-CD137 bispecific antibody of the present invention binds to human CLDN4 and human CD137. It can be checked by employing a known binding activity measurement method whether or not the antibody binds to human CLDN4 or human CD137. Examples of a method for measuring binding activity include a method such as enzyme-linked immunosorbent assay (ELISA) or flow cytometry.

The anti-CLDN4/anti-CD137 bispecific antibody of the present invention can be produced by those skilled in the art based on sequence information and the like of the heavy chain variable regions and the light chain variable regions of the anti-CLDN4 antibody and the anti-CD137 antibody disclosed herein by a method known in this field. In the anti-CLDN4/anti-CD137 bispecific antibody of the present invention, the heavy chain variable regions and the light chain variable regions of the anti-CLDN4 antibody and the anti-CD137 antibody may be those derived from a human antibody, or derived from a humanized antibody, or a combination of these. In production of a humanized antibody, a back mutation may be appropriately introduced using a method well known to those skilled in the art (Bioinformatics, 2015: Vol. 31: p. 434-435). The anti-CLDN4/anti-CD137 bispecific antibody of the present invention can be produced in accordance with, for example, a method described in PCT/JP2022/18350, although not especially limited thereto. Methods for production of an anti-CLDN4/anti-CD137 bispecific antibody described in PCT/JP2022/18350 (including, but not limited to, <Polynucleotide of Bispecific Antibody of Invention>, <Expression Vector of Bispecific Antibody of Invention>, <Host Cell of Invention>, <Method for Producing Bispecific Antibody of Invention> and Examples) are incorporated herein by reference (Incorporation by Reference).

### <Pharmaceutical Composition of Invention>

The pharmaceutical composition of the invention, which is produced using the anti-CLDN4/anti-CD137 bispecific antibody of the present invention, comprises an anti-CLDN4/anti-CD137 bispecific antibody of the present invention and a pharmaceutically acceptable excipient. The pharmaceutical composition of the present invention can be prepared by a usually employed method with an excipient usually used in this field, namely, a pharmaceutical excipient, a pharmaceutical carrier or the like. Examples of a dosage form of such a pharmaceutical composition include parenteral agents such as an injection and a drop, and administration can be performed by an appropriate method such as intravenous administration, subcutaneous administration, or intraperitoneal administration. In formulation, an excipient, a carrier, an additive or the like suitable to the dosage form can be used in a pharmaceutically acceptable range. The pharmaceutical composition of the present invention also comprises an anti-CLDN4/anti-CD137 bispecific antibody of the present invention and a pharmaceutically acceptable excipient, as described above, but may further contain a PD-1 signal inhibitor in one embodiment.

The pharmaceutical composition of the present invention can contain a post-translationally modified product of the anti-CLDN4/anti-CD137 bispecific antibody of the present invention. For example, a pharmaceutical composition comprising an antibody or the like comprising both of or one of lysine deletion in the C-terminal and pyroglutamylation in the N-terminal can be embraced in the present invention.

In one embodiment, the pharmaceutical composition of the present invention is a pharmaceutical composition comprising an anti-CLDN4/anti-CD137 bispecific antibody comprising: a heavy chain variable region of an anti-CLDN4 antibody comprising a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and a light chain variable region of an anti-CLDN4 antibody comprising a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4, a heavy chain variable region of an anti-CD137 antibody comprising a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and a light chain variable region of an anti-CD137 antibody comprising a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2; and/or a post-translationally modified product of the bispecific antibody.

In one embodiment, the pharmaceutical composition of the present invention is a pharmaceutical composition comprising an anti-CLDN4/anti-CD137 bispecific antibody comprising: a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region consisting of an amino acid sequence at amino acid positions 1-109 of SEQ ID NO: 4, and an anti-CD137 scFv comprising a light chain variable region of an anti-CD137 antibody consisting of an amino acid sequence at amino acid positions 464-573 of SEQ ID NO: 2 and a heavy chain variable region of an anti-CD137 antibody consisting of an amino acid sequence at amino acid positions 595-712 of SEQ ID NO: 2, wherein an amino terminal of the anti-CD137 scFv is linked to a heavy chain carboxy terminal of the anti-CLDN4 antibody via a linker; and/or a post-translationally modified product of the bispecific antibody.

In one embodiment, the pharmaceutical composition of the invention is a pharmaceutical composition comprising: an anti-CLDN4/anti-CD137 bispecific antibody comprising a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO 4, and/or a post-translationally modified product of the bispecific antibody.

The amounts of the anti-CLDN4/anti-CD137 bispecific antibody of the present invention added in the formulation vary depending on the degree of symptom and the age of a patient, a dosage form of the formulation to be used, a binding titer of the antibody, or the like, and for example, about 0.0001 mg/kg to 1000 mg/kg of the anti-CLDN4/anti-CD137 bispecific antibody in terms of human dosage can be used in the formulation. In one embodiment, the amount of the anti-CLDN4/anti-CD137 bispecific antibody of the present invention added in formulation is in the range of 0.0001 mg/kg to 1000 mg/kg in terms of human dosage. In one embodiment, the amount of the anti-CLDN4/anti-CD 137 bispecific antibody of the present invention added in formulation is in the range of 0.001 mg/kg to 100 mg/kg in terms of human dosage. In one embodiment, the amount of the anti-CLDN4/anti-CD137 bispecific antibody of the present invention added in formulation is in the range of 0.01 mg/kg to 10 mg/kg in terms of human dosage. In one embodiment, the amount of the anti-CLDN4/anti-CD137 bispecific antibody of the present invention added in formulation is preferably in the range of 0.01 mg/kg to 10 mg/kg in terms of human dosage.

### <PD-1 Signal Inhibitor>

In the present invention, a PD-1 signal inhibitor is used in combination with the anti-CLDN4/anti-CD137 bispecific antibody of the present invention or the pharmaceutical composition of the present invention to treat cancer of a subject.

The mechanism of action and therapeutic modality of the PD-1 signal inhibitor are not particularly limited as long as they block a PD-1 signal. The mechanism of action may be, for example, inhibition of binding between molecules involved in the PD-1 signal, reduction in the expression level of PD-1 signal molecules (for example, inhibition of protein formation or induction of protein degradation), or the like. The therapeutic modality may be, for example, an antibody, a low molecular weight compound, a nucleic acid (which may include DNA or RNA, or a natural or artificial nucleic acid), a fusion protein, a peptide, or other therapeutic modality.

The PD-1 signal inhibitor can be obtained by a method of measuring the inhibitory action on binding of one or more proteins selected from the group consisting of PD-1 and PD-L1 or PD-L2, the expression reducing action using the expression level of PD-1 signal molecules or the like as an indicator, and the like. For example, an inhibitor of binding of PD-1 with PD-L1 or PD-L2 may be obtained by obtaining inhibitors that bind to PD-1 and PD-L1 or PD-L2, and then screening the obtained inhibitors for their ability to inhibit binding between PD-1 and PD-L1 or PD-L2. The binding of an inhibitor to a protein can be evaluated using methods well known to those skilled in the art, such as flow cytometry (FCM), ELISA, surface plasmon resonance (SPR), thermal shift assay (TSA), and isothermal titration calorimetry (ITC). For example, an inhibitor that reduces the expression level of PD-1 signal molecules such as PD-1, PD-L1, or PD-L2 can be obtained by using the protein level of PD-1, PD-L1, or PD-L2 in cells as an indicator. The inhibitory action of the PD-1 signal can be confirmed by an action such as T cell proliferation IFN-y release or a reporter assay. The action of an inhibitor to reduce the expression level of a certain protein can be confirmed using a method well known to those skilled in the art, for example, ELISA, quantitative PCR, in situ hybridization, and live cell imaging.

Examples of the PD-1 signal inhibitor include an antibody inhibiting a PD-1 signal such as an anti-PD-1 antibody, an anti-PD-L1 antibody, or an anti-PD-L2 antibody. Such an antibody may be a humanized antibody, a chimeric antibody, a mouse antibody, a human antibody, and an antigen binding fragment thereof. For example, known anti-PD-1 antibodies include, but are not limited to, the antibodies described in U.S. Patent No. 8008449, U.S. Patent No. 6808710, U.S. Patent No. 7488802, U.S. Patent No. 8168757, U.S. Patent No. 8354509, WO2006/121168, and WO2012/145493. For example, known anti-PD-L1 antibodies include, but are not limited to, antibodies described in WO2007/005874, WO2010/077634, WO2011/066389, WO2013/079174, and U.S. Patent No. 8217149. In one embodiment, the PD-1 signal inhibitor used in the present invention is an anti-PD-1 antibody, an anti-PD-L1 antibody, or an anti-PD-L2 antibody, or an antigen binding fragment thereof. In one embodiment, the PD-1 signal inhibitor used in the present invention is an anti-PD-1 antibody, an anti-PD-L1 antibody, or an anti-PD-L2 antibody. In one embodiment, the anti-PD-1 antibody may be an anti-PD-1 antibody such as nivolumab, pembrolizumab, pidilizumab, spartalizumab, and cemiplimab. In one embodiment, the anti-PD-L1 antibody may be an anti-PD-L1 antibody, such as atezolizumab, durvalumab, and avelumab.

Examples of the PD-1 signal inhibitor include a fusion protein and a low molecular weight compound that inhibit the binding of PD-1 to PD-L1, such as AMP-224 (WO2010/027827 and WO2011/066342) and BMS-1166 (Oncotarget, 2017: Vol. 8: p. 72167-72181). Various PD-1 signal inhibitors are known in the art (NPL 8).

### <Therapeutic Method of Invention>

The therapeutic method of the present invention is a method for treating cancer, including administering an anti-CLDN4/anti-CD137 bispecific antibody of the present invention and a PD-1 signal inhibitor to a subject (referred to as the "therapeutic method of the present invention").

In one embodiment, the therapeutic method of the present invention is characterized in that the anti-CLDN4/anti-CD137 bispecific antibody of the present invention is used (administered) in combination with a PD-1 signal inhibitor to the subject simultaneously, continuously, or sequentially.

In one embodiment, the therapeutic method of the present invention is characterized in that the anti-CLDN4/anti-CD137 bispecific antibody of the present invention and the PD-1 signal inhibitor are (i) comprised in the same pharmaceutical composition and administered simultaneously or (ii) comprised in separate pharmaceutical compositions and used in combination simultaneously, continuously, or sequentially, to the subject.

In one embodiment, the therapeutic method of the present invention is characterized in that the anti-CLDN4/anti-CD137 bispecific antibody of the present invention and the PD-1 signal inhibitor are (i) comprised in the same pharmaceutical composition and administered simultaneously or (ii) comprised in separate pharmaceutical compositions and administered to a subject on the same day, to the subject.

In one embodiment, the therapeutic method of the present invention is characterized by continuous use of (a) starting administration of a PD-1 signal inhibitor after completion of administration of the anti-CLDN4/anti-CD137 bispecific antibody of the present invention to the subject or (b) starting administration of the anti-CLDN4/anti-CD137 bispecific antibody of the present invention after completion of administration of the PD-1 signal inhibitor to the subject.

In one embodiment, the method of treatment of the present invention is characterized by sequential use of administering the anti-CLDN4/anti-CD137 bispecific antibody and the PD-1 signal inhibitor of the present invention to a subject according to a dosage regimen that includes a dosing cycle. In one embodiment, the therapeutic method of the present invention is characterized in that administration of the anti-CLDN4/anti-

CD137 bispecific antibody or the pharmaceutical composition of the present invention to the subject can be initiated in at least one or all dosing cycles, followed by administration of the PD-1 signal inhibitor. In one embodiment, the therapeutic method of the present invention is characterized in that administration of the PD-1 signal inhibitor to the subject can be initiated in at least one or all dosing cycles, followed by administration of the anti-CLDN4/anti-CD137 bispecific antibody or the pharmaceutical composition of the present invention.

### <Therapeutic Use>

Cancer treated by the pharmaceutical composition, therapeutic method, and the like of the present invention may be either solid cancer or blood cancer. Cancer treated by the present invention may be either primary or metastatic. Cancer treated by the present invention is not especially limited, and examples thereof include various peritoneal metastatic cancers, gastric cancer, lung cancer, blood cancers such as acute lymphoblastic leukemia, acute myelogenous leukemia, Hodgkin's lymphoma, non-Hodgkin's lymphoma, B cell lymphoma, multiple myeloma and T cell lymphoma, solid cancers such as myelodysplastic syndromes, adenocarcinoma, squamous cell carcinoma, adenosquamous carcinoma, undifferentiated carcinoma, large cell carcinoma, non-small cell lung cancer, small cell lung cancer, mesothelioma, skin cancer, skin T cell lymphoma, breast cancer, prostate cancer, bladder cancer, vaginal cancer, cervix cancer, head and neck cancer, uterine cancer, cervical cancer, liver cancer, gallbladder cancer, bile duct cancer, kidney cancer, pancreatic cancer, colon cancer, colorectal cancer, rectal cancer, small intestine cancer, gastric cancer, esophageal cancer, testicular cancer, ovarian cancer and brain tumor, cancers of bone tissues, cartilage tissues, adipose tissues, muscle tissues, vascular tissues and blood-forming tissues, sarcomas such as chondrosarcoma, Ewing's sarcoma, malignant hemangioendothelioma, malignant schwannoma, osteosarcoma and soft tissue sarcoma, and blastomas such as glioblastoma, glioblastoma multiforme, hepatoblastoma, medulloblastoma, nephroblastoma, neuroblastoma, pancreatoblastoma, pleuropulmonary blastoma and retinoblastoma.

In one embodiment, cancer to be treated by the present invention is colorectal cancer, non-small cell lung cancer, small cell lung cancer, bladder cancer, ovarian cancer, breast cancer or prostate cancer. In one embodiment, cancer to be treated by the present invention is cancer in which CLDN4 is highly expressed as compared to a normal tissue. Cancer to be treated by the present invention is preferably cancer in which CLDN4 is highly expressed as compared to a normal tissue, or cancer selected from the group consisting of colorectal cancer, rectal cancer, lung cancer, non-small cell lung cancer, small cell lung cancer, bladder cancer, ovarian cancer, breast cancer and prostate cancer.

The dosage of the anti-CLDN4/anti-CD137 bispecific antibody of the present invention or the PD-1 signal inhibitor to a subject varies depending on the degree of symptom or age of the subject, a dosage form of an antibody, a pharmaceutical composition, an inhibitor, and the like to be used, or activity strength of the active ingredient or the like, and they can be used in an amount of, for example, about 0.0001 mg/kg to 1000 mg/kg. In one embodiment, the anti-CLDN4/anti-CD137 bispecific antibody of the present invention is administered to a subject at a dose of 0.0001 mg/kg to 1000 mg/kg. In one embodiment, the anti-CLDN4/anti-CD137 bispecific antibody of the present invention is administered to a subject at a dose of 0.001 mg/kg to 100 mg/kg. In one embodiment, the anti-CLDN4/anti-CD137 bispecific antibody of the present invention is administered to a subject at a dose of 0.01 mg/kg to 10 mg/kg.

Specific examples to be referred to for further understanding of the present invention will now be provided, and it is noted that these examples are merely illustrative and do not limit the present invention.

### EXAMPLES

### [Example 1: Production of Anti-CLDN4/anti-CD137 Bispecific Antibody]

### [Example 1-1: Production of Vector Encoding Anti-CLDN4/anti-CD137 Bispecific Antibody]

It has been reported that KM3900 that is an anti-CLDN4 antibody binds selectively to CLDN4 as compared with other claudin family molecules such as CLDN6 (PTL 1). Therefore, a humanized antibody of KM3900 was produced based on this report.

Specifically, based on a humanized amino acid sequence of a variable region of KM3900, a humanized antibody was designed from sequences of a human Igγ1 constant region and a human Igκ constant region. Amino acid mutations L234A, L235A, and P331G were introduced into the human Igγ1 constant region to design an anti-CLDN4 antibody sequence. The thus designed humanized anti-CLDN4 antibody is referred to as "hKM3900."

AlivaMab mice (Ablexis, U.S. Patent No. 9346873) were immunized by administering the human CD137-human Fc fusion protein described in Example 3 of PCT/JP2022/18350 and immunoadjuvants several times. By a conventional method, Lymphocytes were collected from the lymph nodes of the immunized mice and were subjected to cellular fusion with mouse myeloma cells SP2/0 to produce hybridomas. Single colonies of the hybridomas were isolated with an automatic picking device to obtain monoclonal hybridoma cells (hereinafter referred to as the "clone"). A clone producing antibody binding to human CD137 was screened to obtain a clone producing an anti-CD137 antibody (hereafter referred to as "A2-32"). Furthermore, a cDNA was synthesized from a cell lysate of this clone, and an antibody nucleotide sequence was identified. The anti-CD137 scFv was designed based on the sequences of the heavy chain variable region and the light chain variable region of the A2-32 thus obtained.

The anti-CLDN4/anti-CD137 bispecific antibody was designed based on the amino acid sequence of hKM3900 and the amino acid sequence of the designed anti-CD137 scFv. In the anti-CLDN4/anti-CD137 bispecific antibody, a GS linker is linked to the heavy chain C-terminal of hKM3900 of the IgG1 type, and the N-terminal of the anti-CD 137 scFv is bound to the C-terminal of the GS linker. The designed anti-CLDN4/anti-CD137 bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO 4. A polynucleotide encoding the designed polypeptide comprising the heavy chain of the anti-CLDN4 antibody and the anti-CD 137 scFv, and a polynucleotide encoding the light chain of the anti-CLDN4 antibody were each prepared and inserted into a pcDNA3.4 TOPO vector (Thermo Fisher Scientific) according to a conventional method. The two vectors thus produced are referred to as the "anti-CLDN4/anti-CD137 bispecific antibody expression vectors."

### [Example 1-2: Production of Anti-CLDN4/anti-CD137 Bispecific Antibody]

The anti-CLDN4/anti-CD137 bispecific antibody expression vectors were used to produce anti-CLDN4/anti-CD137 bispecific antibodies. Specifically, the anti-CLDN4/anti-CD137 bispecific antibody expression vectors were introduced into ExpiCHO-S cells (Thermo Fisher Scientific, A29127) with ExpiFectamine CHO Transfection Kit (Thermo Fisher Scientific, A29129) to cause the anti-CLDN4/anti-CD137 bispecific antibodies to be secreted into a culture supernatant. From the culture supernatant thus obtained, an anti-CLDN4/anti-CD137 bispecific antibody, hKM3900_tA2-32LH, was purified by affinity purification with MabSelect SuRe (Cytiva, 17-5438-02), followed by size exclusion chromatography purification with HiLoad 26/600 superdex 200 pg (GE Healthcare, 28-9893-36).

The anti-CLDN4/anti-CD137 bispecific antibody hKM3900_tA2-32LH produced in Example 1 is referred to as the "anti-CLDN4/anti-CD137 bispecific antibody" in some cases.

### [Example 2: in vitro Combination Use Effect of Anti-CLDN4/anti-CD137 Bispecific Antibody and Anti-PD-1 Antibody]

*In vitro* combination use effects of the anti-CLDN4/anti-CD137 bispecific antibody and the anti-PD-1 antibody were examined in a coculture system using the human OKT3 scFv expressing LCLC-103H cells and expanded pan T cells.

### [Example 2-1: Preparation of Expanded Pan T Cell]

One obtained by adding FBS (Cytiva, SH30084.03) in a final concentration of 10% and penicillin streptomycin (Thermo Fisher Scientific, 15070-063) in a final concentration of 1% to RPMI-1640 (Sigma, R8758) is referred to as the "culture medium." An anti-CD3 antibody (BioLegend, 317325) was added to a 150 mm/Tissue Culture Dish (IWAKI, 3030-150) (hereinafter referred to as the "dish") at a final concentration of 1 µg/mL, and the anti-CD3 antibody was immobilized. Pan T Cell Isolation Kit, human (Miltenyi Biotec, 130-096-535) was used to isolate Pan T cells (including both CD4-positive T cells and CD8-positive T cells; hereinafter referred to as the "Pan T cells") from human peripheral blood mononuclear cells (LONZA, CC-2702) in accordance with a protocol recommended by the manufacturer. The isolated Pan T cells were centrifuged, a supernatant was removed, and the resultant was suspended in the culture medium. The whole Pan T cells suspended in the culture medium were seeded in the dish in which the anti-CD3 antibody had been immobilized. Besides, human IL-2 (PeproTech, 200-2) in a final concentration of 200 U/mL and an anti-CD28 antibody (BioLegend, 302934) in a final concentration of 4 µg/mL were added thereto, and the resultant was cultured at 37°C in a 5% CO₂ incubator. The Pan T cells were collected 3 days after the start of culture, the collected Pan T cells were suspended in the culture medium and seeded in a dish. Furthermore, human IL-2 in a final concentration of 200 U/mL was added thereto, and the resultant was cultured at 37°C in a 5% CO₂ incubator. Four days later, the whole Pan T cells were collected to be centrifuged, a supernatant was removed, the resultant was suspended again in a Cell Banker (Takara, CB011), and the resultant was separated into tubes to be cryopreserved at -80°C. The Pan T cells thus cryopreserved are herein referred to as the "expanded Pan T cells."

### [Example 2-2: Acquisition of Human CD3 Antibody Single-Chain Variable Fragment (OKT3 scFv) Expressing LCLC-103H Cell]

LCLC-103H cells of a human large cell lung cancer cell line expressing human CLDN4 were obtained from Deutsche Sammlung von Mikroorganismen und Zellkulturen (DSMZ, ACC384). The LCLC-103H cells of a human large cell lung cancer cell line were cultured in culture medium under conditions of 37°C and 5% CO₂. A polynucleotide encoding human OKT3 scFv (Journal of Immunological Methods, 2010: 362: p. 131-141) produced according to gene synthesis by a conventional method was subcloned into a **pcDNA3.4** TOPO vector. The produced human OKT3 scFv expression vector was lipofected into LCLC-103H cells using Lipofectamine LTX (Invitrogen, 15338-100) in accordance with a protocol recommended by the manufacturer. LCLC-103H cell clones stably expressing human OKT3 scFv (hereinafter referred to as the "LCLC-OKT3scFv cells") were obtained by selective culture in a culture medium where Geneticin Selective Antibiotic (Thermo Fisher Scientific, 10131-027) was added at a final concentration of 600 µg/mL and limiting dilution.

### [Example 2-3: Combination use Effect of Anti-CLDN4/anti-CD137 Bispecific Antibody and Anti-PD-1 Antibody on in vitro Interferon-y Production Promoting Activity in Coculture System of Cancer Cell and T Cell]

The T cell activity enhancing activity of the anti-CLDN4/anti-CD137 bispecific antibody and the PD-1 antibody was evaluated in a coculture system of the LCLC-OKT3 scFv cells and the expanded Pan T cells using the interferon-y production promoting function as an indicator. The LCLC-OKT3 scFv cells were prepared at 2 × 10⁵ cells/mL in the culture medium and seeded in a flat bottom 96 well plate (IWAKI, 4020-010) at 50 µL each to be cultured at 37°C in a 5% CO₂ incubator. On the next day, the expanded Pan T cells prepared at 1.32 × 10⁶ cells/mL in the culture medium were seeded in a flat bottom 96 well plate in culture at 30 µL each. The hKM3900_tA2-32LH and anti-human PD-1 antibody nivolumab obtained in Example 1 were used as test antibodies. The amino acid sequence of nivolumab was designed based on the amino acid sequences of the heavy chain and the light chain of nivolumab described in WO2014/055648. Nivolumab was obtained from the designed amino acid sequence according to the method described in Example 11 of WO2021/241616. As the isotype control (referred to as the "isotype" in Fig. 1-1), an anti-lysozyme antibody was prepared and used. As a single treatment condition, hKM3900_tA2-32LH serially diluted from a maximum concentration of 50000 ng/mL with a common ratio of about 3 or nivolumab serially diluted from a maximum concentration of 50000 ng/mL with a common ratio of about 3 was added to the culture medium. As a combination use condition, hKM3900_tA2-32LH serially diluted from a maximum concentration of 50000 ng/mL with a common ratio of about 3 was added at 20 µL each to a culture medium comprising nivolumab at a concentration of 50000 ng/mL. The nivolumab after the addition has a final concentration of 10 µg/mL. After the addition, the resultant was cultured at 37°C in a 5% CO₂ incubator. After 4 days, a production amount of interferon y in a culture supernatant was measured with AlphaLISA Interferon-y Measurement Kit (Perkin Elmer, AL217C) in accordance with a protocol recommended by the manufacturer. Fig. 1-1 illustrates the production amount of interferon y. The average and standard deviation were calculated for each condition. hKM3900_tA2-32LH and nivolumab exhibited interferon-y production promoting activity in a coculture system of the human CLDN4 expressing cancer cell line and the expanded Pan T cells. Combination use of hKM3900_tA2-32LH and nivolumab induced stronger interferon-y production promoting activity than a single treatment of either hKM3900_tA2-32LH or nivolumab alone.

### [Example 3: in vitro Combination use Effect of Anti-CLDN4/anti-CD137 Bispecific Antibody and Anti-PD-L1 Antibody]

Based on the sequence of the anti-PD-L1 antibody, atezolizumab antibody, described WO2012/155019 (SEQ ID NOs: 22 and 23), an anti-PD-L1 antibody atezolizumab analog was designed using the sequence of the human Igγ1 constant region with mutations in the Fc region (hereafter referred to as "atezolizumab analog") to obtain an antibody according to the method described in Example 4-2 of PCT/JP2022/18350. *In vitro* combination use effects of hKM3900_tA2-32LH and atezolizumab analog were examined in a coculture system using the LCLC-OKT3 scFv cells and expanded pan T cells obtained in Example 2. Specifically, the T cell activity enhancing activity of the hKM3900_tA2-32LH and the atezolizumab analog was evaluated in a coculture system of the LCLC-OKT3 scFv cells and the expanded Pan T cells using the production amount of interferon y as an indicator. The LCLC-OKT3 scFv cells were prepared at 2 × 10⁵ cells/mL in the culture medium and seeded in a flat bottom 96 well plate at 50 µL each to be cultured at 37°C in a 5% CO₂ incubator. On the next day, the expanded Pan T cells prepared at 1.33 × 10⁶ cells/mL in the culture medium were seeded in a flat bottom 96 well plate in culture at 30 µL each. The hKM3900_tA2-32LH and atezolizumab analog obtained in Example 1 were used as test antibodies. As the isotype control (referred to as the "isotype" in Fig. 1-2), an anti-lysozyme antibody was prepared and used. As a single treatment condition, hKM3900_tA2-32LH serially diluted from a maximum concentration of 50000 ng/mL with a common ratio of about 3 or atezolizumab analog serially diluted from a maximum concentration of 50000 ng/mL with a common ratio of about 3 was added to the culture medium. As a combination use condition, hKM3900_tA2-32LH serially diluted from a maximum concentration of 50000 ng/mL with a common ratio of about 3 was added at 20 µL each to a culture medium comprising atezolizumab analog at a concentration of 50000 ng/mL. The atezolizumab analog after the addition has a final concentration of 10 µg/mL. After the addition, the resultant was cultured at 37°C in a 5% CO₂ incubator. After 5 days, a production amount of interferon y in a culture supernatant was measured with AlphaLISA Interferon-y Measurement Kit (Perkin Elmer, AL217C) in accordance with a protocol recommended by the manufacturer. Fig. 1-2 illustrates the production amount of interferon y. The average and standard deviation were calculated for each condition. hKM3900_tA2-32LH and atezolizumab analog exhibited interferon-y production promoting activity in a coculture system of the human CLDN4 expressing cancer cell line and the expanded Pan T cells. Combination use of hKM3900_tA2-32LH and atezolizumab analog induced stronger interferon-y production promoting activity than a single treatment of either hKM3900_tA2-32LH or atezolizumab analog alone.

### [Example 4: in vivo Combination use Effect of Anti-CLDN4/anti-CD137 Bispecific Antibody and Anti-mouse PD-1 Antibody]

*In vivo* antitumor effects of the anti-CLDN4/anti-CD137 bispecific antibody and the anti-human PD-1 antibody were examined using B-h4-1BB mice (human CD137 knock-in mice) transplanted with human CLDN4 expressing B16-F10 cells.

### [Example 4-1: Construction of Human CLDN4 Expressing B16-F10 Cell]

The murine melanoma cell line B16-F10 cells were obtained from the American Type Culture Collection (ATCC, CRL-6475). The cells were cultured in a Dulbecco's modified Eagle medium (Sigma, D6429) where a final concentration of 10% inactivated fetal bovine serum (FBS) (Cytiva, SH30084.03) was added (the medium after preparation is hereafter referred to as the "Eagle's culture medium") under conditions of 37°C and 5% CO₂. CLDN4 (Myc-DDK-tagged)-Human claudin 4 (CLDN4) (ORIGENE, RC200490) was introduced into B16-F10 cells with jetPRIME (Polyplus-transfection, 114-15). B16-F10 cell clones stably expressing human CLDN4 (hereafter referred to as "human CLDN4 expressing B16-F10 cells") were obtained by selection in the Eagle's culture medium where G418 (Nacalai Tesque, Inc., 09380-44) was added at a final concentration of 1 mg/mL.

### [Example 4-2: Combination use Effect of Anti-CLDN4/anti-CD137 Bispecific Antibody and Anti-mouse PD-1 Antibody on in vivo Antitumor Activity]

B-h4-1BB male mice (C57BL/6-Tnfrsf9^{tm1 (Tnfrsf9)}/Bcgen; Biocytogen, 110004) (hereafter referred to as "mice") were obtained and bred. A human CLDN4 expressing B16-F10 cell was suspended in PBS (-) (WAKO, 045-29795), and a 4 × 10⁶ cells/mL cell suspension was prepared. The cell suspension was inoculated subcutaneously into the back of 6-week-old mice at 2 × 10⁵ cells/50 µL each. Tumor diameter was measured using a caliper (Mitutoyo Corporation, CD-15AXR) 3 days after cell inoculation. For calculation of tumor volume [mm³], the following formula was used. [Tumor volume (mm3)] = [Major tumor diameter (mm)] × [Minor tumor diameter(mm)]2 × 0.5

The cell-inoculated mice were divided into groups (n = 10) so that the tumor volume was equal in each group to start administration of the test antibodies. The first day of the administration was defined as day 0. The anti-CLDN4/anti-CD137 bispecific antibody hKM3900_tA2-32LH obtained in Example 1 was used as the test antibody, and an anti-mouse PD-1 antibody (Bio X Cell, BE0146) was used as the anti-PD-1 antibody. An anti-lysozyme antibody was used as an isotype control antibody for hKM3900_tA2-32LH, and a rat IgG2a isotype control antibody (Bio X Cell, BE0089) (referred to as a "control" in Fig. 2) was used as an isotype control antibody for an anti-mouse PD-1 antibody. Details of the test antibodies administered to the four tested groups, the dose, and the administration schedule are provided below.

### (1) Group 1: Control Group

An anti-lysozyme antibody was intraperitoneally administered at 0.3 mg/kg on days 0 and 7, and a rat IgG2a isotype control antibody was intraperitoneally administered at 100 µg/head on days 0, 4, 7, and 11.

### (2) Group 2: hKM3900_tA2-32LH Administration Group

hKM3900_tA2-32LH was intraperitoneally administered at 0.3 mg/kg on days 0 and 7, and a rat IgG2a isotype control antibody was intraperitoneally administered at 100 µg/head on days 0, 4, 7, and 11.

### (3) Group 3: Anti-mouse PD-1 Antibody Administration Group

An anti-lysozyme antibody was intraperitoneally administered at 0.3 mg/kg on days 0 and 7, and an anti-mouse PD-1 antibody was intraperitoneally administered at 100 µg/head on days 0, 4, 7, and 11.

### (4) Group 4: hKM3900_tA2-32LH + Anti-mouse PD-1 Antibody Combined Administration Group

hKM3900_tA2-32LH was intraperitoneally administered at 0.3 mg/kg on days 0 and 7, and an anti-mouse PD-1 antibody was intraperitoneally administered at 100 µg/head on days 0, 4, 7, and 11.

Tumor volume was assessed on days 4, 7, 11, and 14 for each group. The tumor volumes on day 14 of Group 2 and Group 3 were compared with that of Group 4 through unpaired Student's t-test (Fig. 2).

As illustrated in Fig. 2, the tumor volume in the combination use group of hKM3900_tA2-32LH and the anti-mouse PD-1 antibody was significantly smaller than that in the hKM3900_tA2-32LH alone administration group and the anti-mouse PD-1 antibody alone administration group. The results suggest that combination useof an anti-CLDN4/anti-CD137 bispecific antibody and an anti-PD-1 antibody possibly has a greater antitumor effect in the treatment of human cancer than administration of either antibody alone.

### INDUSTRIAL APPLICABILITY

A method for treating cancer using an anti-CLDN4/anti-CD 137 bispecific antibody of the present invention in combination with a PD-1 signal inhibitor is expected to be useful for treatment of cancer.

### SEQUENCE LISTING FREE TEXT

SEQ ID NO: 2 shows an amino acid sequence of hKM3900_tA2-32LH HC, and the nucleotide sequence set forth in SEQ ID NO: 1 shows a nucleotide sequence encoding an amino acid sequence of a heavy chain of hKM3900_tA2-32LH set forth in SEQ ID NO: 2. SEQ ID NO: 4 shows an amino acid sequence of hKM3900 LC, and the nucleotide sequence set forth in SEQ ID NO: 3 shows a nucleotide sequence encoding an amino acid sequence of a light chain of hKM3900 set forth in SEQ ID NO: 4. SEQ ID NOS: 5 to 14 show amino acid sequences of the various linkers described in the detailed description of the invention.

## Claims

1. A pharmaceutical composition for treating cancer of a subject, comprising an anti-CLDN4/anti-CD137 bispecific antibody, which is used in combination with a PD-1 signal inhibitor, wherein the bispecific antibody comprises a heavy chain variable region and a light chain variable region of an anti-CLDN4 antibody, and a heavy chain variable region and a light chain variable region of an anti-CD137 antibody.

2. The pharmaceutical composition according to claim 1, wherein the heavy chain variable region of the anti-CLDN4 antibody comprises a CDR1 consisting of an amino acid sequence at amino acid positions 31-35 of SEQ ID NO: 2, a CDR2 consisting of an amino acid sequence at amino acid positions 50-66 of SEQ ID NO: 2, and a CDR3 consisting of an amino acid sequence at amino acid positions 99-112 of SEQ ID NO: 2, and the light chain variable region of the anti-CLDN4 antibody comprises a CDR1 consisting of an amino acid sequence at amino acid positions 24-35 of SEQ ID NO: 4, a CDR2 consisting of an amino acid sequence at amino acid positions 51-57 of SEQ ID NO: 4, and a CDR3 consisting of an amino acid sequence at amino acid positions 90-98 of SEQ ID NO: 4.

3. The pharmaceutical composition according to claim 1 or 2, wherein the heavy chain variable region of the anti-CLDN4 antibody consists of an amino acid sequence at amino acid positions 1-123 of SEQ ID NO: 2, and the light chain variable region of the anti-CLDN4 antibody consists of an amino acid sequence at amino acid positions 1-109 of SEQ ID NO: 4.

4. The pharmaceutical composition according to any one of claims 1 to 3, wherein the anti-CLDN4/anti-CD137 bispecific antibody comprises an IgG antibody (anti-CLDN4 IgG antibody) consisting of a heavy chain comprising the heavy chain variable region of the anti-CLDN4 antibody and a light chain comprising the light chain variable region of the anti-CLDN4 antibody.

5. The pharmaceutical composition according to claim 4, comprising either LALA mutation (L234A and L235A) or P331G mutation (wherein a position of the mutation is an amino acid position in human Igγ1 constant region according to EU index) or both in a Fc region of the anti-CLDN4 IgG antibody.

6. The pharmaceutical composition according to any one of claims 1 to 5, wherein the heavy chain variable region of the anti-CD137 antibody comprises a CDR1 consisting of an amino acid sequence at amino acid positions 625-629 of SEQ ID NO: 2, a CDR2 consisting of an amino acid sequence at amino acid positions 644-659 of SEQ ID NO: 2, and a CDR3 consisting of an amino acid sequence at amino acid positions 692-701 of SEQ ID NO: 2, and the light chain variable region of the anti-CD 137 antibody comprises a CDR1 consisting of an amino acid sequence at amino acid positions 486-498 of SEQ ID NO: 2, a CDR2 consisting of an amino acid sequence at amino acid positions 514-520 of SEQ ID NO: 2, and a CDR3 consisting of an amino acid sequence at amino acid positions 553-563 of SEQ ID NO: 2.

7. The pharmaceutical composition according to any one of claims 1 to 6, wherein the heavy chain variable region of the anti-CD137 antibody consists of an amino acid sequence at amino acid positions 595-712 of SEQ ID NO: 2, and the light chain variable region of the anti-CD137 antibody consists of an amino acid sequence at amino acid positions 464-573 of SEQ ID NO: 2.

8. The pharmaceutical composition according to claim 6 or 7, wherein the anti-CLDN4/anti-CD137 bispecific antibody comprises an anti-CD137 single chain variable fragment (anti-CD137 scFv) comprising the heavy chain variable region and the light chain variable region of the anti-CD137 antibody.

9. The pharmaceutical composition according to claim 8, wherein the anti-CD137 scFv consists of an amino acid sequence at amino acid positions 464-712 of SEQ ID NO: 2.

10. The pharmaceutical composition according to claim 8 or 9, wherein the anti-CLDN4/anti-CD137 bispecific antibody comprises an anti-CLDN4 IgG antibody and the anti-CD137 scFv, and an amino terminal of the anti-CD 137 scFv is linked to a heavy chain carboxy terminal of the anti-CLDN4 IgG antibody via a linker.

11. A pharmaceutical composition for treating cancer of a subject, comprising an anti-CLDN4/anti-CD137 bispecific antibody, which is used in combination with a PD-1 signal inhibitor, wherein the bispecific antibody comprises a heavy chain of an anti-CLDN4 antibody comprising a heavy chain variable region consisting of an amino acid sequence at amino acid positions 1-123 of SEQ ID NO: 2 and a light chain of an anti-CLDN4 antibody comprising a light chain variable region consisting of an amino acid sequence at amino acid positions 1-109 of SEQ ID NO: 4, and an anti-CD137 scFv comprising a light chain variable region of an anti-CD137 antibody consisting of an amino acid sequence at amino acid positions 464-573 of SEQ ID NO: 2 and a heavy chain variable region of an anti-CD137 antibody consisting of an amino acid sequence at amino acid positions 595-712 of SEQ ID NO: 2, wherein an amino terminal of the anti-CD137 scFv is linked to a heavy chain carboxy terminal of the anti-CLDN4 antibody via a linker.

12. A pharmaceutical composition for treating cancer of a subject, comprising an anti-CLDN4/anti-CD137 bispecific antibody, which is used in combination with a PD-1 signal inhibitor, wherein the bispecific antibody comprises a heavy chain of an anti-CLDN4 antibody consisting of an amino acid sequence at amino acid positions 1-453 of SEQ ID NO: 2 and a light chain of an anti-CLDN4 antibody consisting of an amino acid sequence at amino acid positions 1-215 of SEQ ID NO: 4, and an anti-CD137 scFv consisting of an amino acid sequence at amino acid positions 464-712 of SEQ ID NO: 2, wherein an amino terminal of the anti-CD137 scFv is linked to a heavy chain carboxy terminal of the anti-CLDN4 antibody via a linker.

13. The pharmaceutical composition according to any one of claims 10 to 12, wherein the linker is a GS linker.

14. A pharmaceutical composition for treating cancer of a subject, comprising an anti-CLDN4/anti-CD137 bispecific antibody, which is used in combination with a PD-1 signal inhibitor, wherein the bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4.

15. The pharmaceutical composition according to any one of claims 1 to 14, wherein the anti-CLDN4/anti-CD137 bispecific antibody is post-translationally modified.

16. The pharmaceutical composition according to any one of claims 1 to 15, which is used in combination with the PD-1 signal inhibitor simultaneously, continuously, or sequentially.

17. The pharmaceutical composition according to any one of claims 1 to 16, wherein the anti-CLDN4/anti-CD137 bispecific antibody and the PD-1 signal inhibitor are (i) comprised in the same pharmaceutical composition and administered simultaneously or (ii) comprised in separate pharmaceutical compositions and used in combination simultaneously, continuously, or sequentially.

18. The pharmaceutical composition according to any one of claims 1 to 17, wherein the cancer is selected from the group consisting of colorectal cancer, bladder cancer, and lung cancer.

19. The pharmaceutical composition according to any one of claims 1 to 18, wherein the PD-1 signal inhibitor is an antibody binding to one or more proteins selected from the group consisting of PD-1, PD-L1, and PD-L2, or an antigen binding fragment thereof.

20. The pharmaceutical composition according to any one of claims 1 to 19, wherein the PD-1 signal inhibitor is an anti-PD-1 antibody selected from the group consisting of nivolumab, pembrolizumab, pidilizumab, spartalizumab, and cemiplimab.

21. The pharmaceutical composition according to any one of claims 1 to 19, wherein the PD-1 signal inhibitor is an anti-PD-L1 antibody selected from the group consisting of atezolizumab, durvalumab, and avelumab.

22. An anti-CLDN4/anti-CD137 bispecific antibody used for treating cancer of a subject, comprising a heavy chain variable region and a light chain variable region of an anti-CLDN4 antibody, and a heavy chain variable region and a light chain variable region of an anti-CD137 antibody, wherein the bispecific antibody is used in combination with a PD-1 signal inhibitor.

23. An anti-CLDN4/anti-CD137 bispecific antibody used for treating cancer of a subject, comprising a bispecific antibody consisting of a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4, wherein the bispecific antibody is used in combination with a PD-1 signal inhibitor.

24. A method for treating cancer, comprising administering an anti-CLDN4/anti-CD137 bispecific antibody and a PD-1 signal inhibitor to a subject, wherein the anti-CLDN4/anti-CD137 bispecific antibody comprises a heavy chain variable region and a light chain variable region of an anti-CLDN4 antibody, and a heavy chain variable region and a light chain variable region of an anti-CD137 antibody.

25. A method for treating cancer, comprising administering an anti-CLDN4/anti-CD137 bispecific antibody in combination with a PD-1 signal inhibitor to a subject, wherein the bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4.

26. Use of an anti-CLDN4/anti-CD137 bispecific antibody for production of a pharmaceutical composition used in combination with a PD-1 signal inhibitor to treat cancer of a subject, wherein the bispecific antibody comprises a heavy chain variable region and a light chain variable region of an anti-CLDN4 antibody, and a heavy chain variable region and a light chain variable region of an anti-CD137 antibody.

27. Use of an anti-CLDN4/anti-CD137 bispecific antibody for production of a pharmaceutical composition used in combination with a PD-1 signal inhibitor to treat cancer of a subject, wherein the bispecific antibody comprises a polypeptide comprising a heavy chain of an anti-CLDN4 antibody and an anti-CD137 scFv, consisting of an amino acid sequence of SEQ ID NO: 2, and a light chain of an anti-CLDN4 antibody consisting of an amino acid sequence of SEQ ID NO: 4.
